# EUROPEAN PATENT APPLICATION

(11) **EP 4 393 943 A1**
(43) Date of publication of application: **03.07.2024**
(21) Application number: 22860650.5
(22) Date of filing: 26.08.2022
(51) Int. Cl.: C07K 14/725, C07K 16/28, C07K 16/30, C07K 19/00, C12N 15/12, C12N 15/62, A61K 39/395, A61P 35/00

(54) **POLYPEPTIDE FUSION MOLECULE CLOSE TO NATURAL MOLECULE**

(30) Priority: 26.08.2021 CN 202110990796
(71) Applicant: Tioc Therapeutics, Ltd., Hangzhou, Zhejiang 310018 (CN)
(72) Inventor: LI, Yi, Hangzhou, Zhejiang 310018 (CN); TIAN, Ye, Hangzhou, Zhejiang 310018 (CN)
(74) Representative: Mewburn Ellis LLP
(86) International application number: PCT/CN2022/115250
(87) International publication number: WO 2023/025304

(57) **Abstract**

Provided is a multi-domain fusion protein molecule close to a native state. The fusion protein is composed of an antibody single-chain molecule without an immune effect and a polypeptide receptor molecule, which specifically binds to pMHC epitope, or component part thereof or fragment thereof. These protein domains close to the natural state can be mixed to form a fusion protein complex molecule having a biological function, and can avoid the risk of immunogenicity caused by introducing artificial flexible peptide chains to link each domain into a single polypeptide chain molecule.

## Description

### Technical field

The present invention relates to the field of biomedical or biopharmaceutical technology and more specifically to a polypeptide fusion molecule that is close to a natural molecule.

### Background

Protein molecules including antibodies, T-cell receptors, enzymes, and various cytokines, etc., perform various physiological functions in cells and organisms. Currently, most biopharmaceutical macromolecules are derived from antibody molecules through various protein engineering techniques. When combining multiple protein domains together, the most commonly used method is to connect the C-terminus of one protein domain to the N-terminus of another domain through a flexible peptide. For example, scFv is a single chain antibody formed by connecting the heavy chain variable region and light chain variable region of the antibody through a flexible peptide. The main function of the flexible peptide is to express the heavy chain and its corresponding light chain into a single long-chain polypeptide molecule, thereby facilitating expression and downstream preparation and processing, etc. However, there is a risk of immunogenicity when the antibody of this structure becomes a drug. Therefore, there is an urgent need for a method to generate molecules close to their natural state to avoid the above risk for this type of biological drug.

### Summary of the invention

The purpose of the present invention is to obtain a multi-domain fusion protein complex molecule close to its natural state. The elements that make up these multi-domain fusion protein complex molecules close to their natural state, such as fusion proteins with structures as shown in Formulas Ia or Ib, element A2, and element B2 can be mixed to form the multi-domain fusion protein complex molecule having biological functions.

In the first aspect of the present invention, it provides a fusion protein which comprises a polypeptide with a structure from the N-terminus to the C-terminus as shown in Formula Ia or Ib:

A1-L-B (Ia);

B-L-A1 (Ib);

wherein,
element A1 is a polypeptide molecule without an immune effect;
element B is a polypeptide receptor molecule or component thereof, or fragment thereof that specifically binds a pMHC epitope;
element L is a flexible linker; wherein the flexible linker is optional;
"-" is a covalent bond.

In some specific embodiments, it discloses a fusion protein, the structure of which from the N-terminus to the C-terminus is as shown in Formula Ia or Ib:

A1-L-B (Ia);

B-L-A1 (Ib);

wherein,
element A1 is a polypeptide molecule without an immune effect;
element B is a polypeptide receptor molecule or component thereof, or fragment thereof that specifically binds a pMHC epitope;
element L is a flexible linker;
"-" is a covalent bond.

In some specific embodiments, the structure of the fusion protein from the N-terminus to the C-terminus is as shown in Formula Ia.

In some specific embodiments, in the fusion protein, the N-terminus of B (a polypeptide receptor molecule or component thereof, or fragment thereof that specifically binds a pMHC epitope) is connected to the C-terminus of A1 (e.g., an antibody single-chain molecule without an immune effect).

In some specific embodiments, the flexible linker is a peptide linker.

In some specific embodiments, the peptide linker has 1-50 amino acids, preferably 1-20 amino acids, preferably 1-10 amino acids, and more preferably 1-6 amino acids.

In some specific embodiments, the peptide linker has the structure (GGGGS)n, wherein n is a positive integer from 1-5.

In some specific embodiments, the peptide linker is GGGGS (SEQ ID No: 9).

In some specific embodiments, the element A1 is selected from a heavy chain variable region of an antibody or a light chain variable region of an antibody.

In some specific embodiments, the antibody has the property of binding T cell surface molecules.

In some specific embodiments, the antibody may be UCHT1, OK3, 12F6, etc., or a mutant molecule of these antibodies, wherein the amino acid sequence of the mutant molecule has at least 95% or more, at least 98% or more, at least 99% or more, at least 99.9% or more identity with the amino acid sequence of the corresponding wild-type molecule.

In some specific embodiments, according to the Kabat numbering system, the amino acid at position 44 of the heavy chain variable region of the antibody is mutated to cysteine (Cys); and the amino acid at position 100 of the light chain variable region of the antibody is mutated to cysteine (Cys).

In some specific embodiments, according to the Kabat numbering system, the amino acid at position 105 of the heavy chain variable region of the antibody is mutated to cysteine (Cys); and the amino acid at position 43 of the light chain variable region of the antibody is mutated to cysteine (Cys).

In some specific embodiments, in the antibody, an amino acid such as glycine (Gly) at position 44 of the heavy chain variable region of the antibody (Kabat numbering system, hereinafter the same) and an amino acid such as glutamine (Gln) at position 100 of the light chain variable region of the antibody are replaced with cysteine (Cys).

In some specific embodiments, in the antibody, an amino acid such as glutamine (Gln) at position 105 of the heavy chain variable region of the antibody and an amino acid such as alanine (Ala) at position 43 of the light chain variable region of the antibody are replaced with cysteine (Cys).

In some specific embodiments, the element A1 does not bind or may bind T cell surface antigens such as CD3 antigens, but does not activate T cells.

In some specific embodiments, the amino acid sequence of the heavy chain variable region of the antibody comprises a sequence as shown in SEQ ID NO: 14, SEQ ID NO: 20, or SEQ ID NO: 5.

In some specific embodiments, the amino acid sequence of the light chain variable region of the antibody comprises a sequence as shown in SEQ ID NO: 16, SEQ ID NO: 22, or SEQ ID NO: 10.

In some specific embodiments, the amino acid sequence of the heavy chain variable region of the antibody is a sequence as shown in SEQ ID NO: 14, SEQ ID NO: 20 or SEQ ID NO: 5.

In some specific embodiments, the amino acid sequence of the light chain variable region of the antibody is a sequence as shown in SEQ ID NO: 16, SEQ ID NO: 22 or SEQ ID NO: 10.

In some specific embodiments, the element B is selected from the group consisting of: a TCR molecule, a single chain αβTCR, a TCRα chain/TCRβ chain heterodimer complex molecule, an antibody molecule Fab complex molecule, a single chain antibody molecule, and combinations thereof.

In some specific embodiments, the element B is composed of a combination of B1 and B2 via a disulfide bond, wherein B1 is a TCRα chain and B2 is a TCRβ chain; or B1 is a TCRβ chain and B2 is a TCRα chain.

In some specific embodiments, the N-terminus of the α or β chain of the heterodimer, or the N-terminus of the single chain αβTCR, or the N-terminus of the TCRα chain, or the N-terminus of the TCRβ chain, or the N-terminus of the heavy or light chain of the antibody molecule Fab, or the N-terminus of the single chain antibody molecule, is linked to the C-terminal amino acid of an antibody single-chain molecule without an immune effect.

In some specific embodiments, the element B (i.e., a polypeptide receptor molecule or component thereof, or fragment thereof that specifically binds a pMHC epitope) of the fusion protein specifically binds pMHC, and the element A1 (i.e., an antibody single-chain molecule without an immune effect) is unable to bind antigen molecules of the T cells, or the element A1 can bind antigen molecules of the T cells but is unable to efficiently induce activation of the T cells or produce a physiological effect.

In some specific embodiments, when the fusion protein is mixed with another free polypeptide domain molecule A2 that has no immune effect, the fusion protein and A2 form a fusion protein-A2 complex molecule, and the fusion protein-A2 complex molecule redirects T cells and can effectively activate the redirected T cells and produce an immunophysiological effect.

In some specific embodiments, the TCR molecules are heterodimer αβTCR polypeptide pairs, wherein the α and β polypeptides each contain TCR variable and constant regions, but do not contain TCR transmembrane and intracellular regions.

In some specific embodiments, the constant regions of the α and β polypeptides of the TCR molecules are connected by a disulfide bond formed by replacing the threonine (Thr) at position 47 in exon 1 of TRAC*01 and the serine (Ser) at position 56 in exon 1 of TRBC*01 or TRBC*02 with a cysteine (Cys).

In some specific embodiments, the constant regions of the α and β polypeptides of the TCR molecules are connected by a disulfide bond formed by replacing the arginine (Arg) at position 52 of the IMTG numbering method in exon 1 of TRAC*01 and the serine (Ser) at position 53 in exon 1 of TRBC*01 or TRBC*02 with a cysteine (Cys).

In some specific embodiments, the constant regions of the α and β polypeptides of the TCR molecules are connected by a disulfide bond formed by replacing the proline (Pro) at position 88 of the IMTG numbering method in exon 1 of TRAC*01 and the alanine (Ala) at position 18 in exon 1 of TRBC*01 or TRBC*02 with a cysteine (Cys).

In some specific embodiments, the constant regions of the α and β polypeptides of the TCR molecules are connected by a disulfide bond formed by replacing the tyrosine (Tyr) at position 9 of the IMTG numbering method in exon 1 of TRAC*01 and the glutamic acid (Glu) at position 19 in exon 1 of TRBC*01 or TRBC*02 with a cysteine (Cys).

In some specific embodiments, the amino acid sequence of the TRAC*01 is shown in SEQ ID NO: 34.

In some specific embodiments, the amino acid sequence of the TRBC*01 is shown in SEQ ID NO: 35.

In some specific embodiments, the amino acid sequence of the TRBC*02 is shown in SEQ ID NO: 36.

In some specific embodiments, the amino acid sequence of the TCRα chain is shown in SEQ ID NO: 1.

In some specific embodiments, the amino acid sequence of the TCRβ chain is shown in SEQ ID NO: 3.

In some specific embodiments, the amino acid sequence of the fusion protein comprises a sequence as shown in SEQ ID NO: 18, SEQ ID NO: 24, SEQ ID NO: 26, SEQ ID NO: 28, SEQ ID NO: 7, SEQ ID NO: 12, or SEQ ID NO: 32, or a sequence that has at least 95% or more, at least 98% or more, at least 99% or more, at least 99.9% or more identity with the sequence as shown in SEQ ID NO: 18, SEQ ID NO: 24, SEQ ID NO: 26, SEQ ID NO: 28, SEQ ID NO: 7, SEQ ID NO: 12, or SEQ ID NO: 32.

In some specific embodiments, the amino acid sequence of the fusion protein is a sequence as shown in SEQ ID NO: 18, SEQ ID NO: 24, SEQ ID NO: 26, SEQ ID NO: 28, SEQ ID NO: 7, SEQ ID NO: 12, or SEQ ID NO: 32, or is a sequence that has at least 95% or more, at least 98% or more, at least 99% or more, at least 99.9% or more identity with the sequence as shown in SEQ ID NO: 18, SEQ ID NO: 24, SEQ ID NO: 26, SEQ ID NO: 28, SEQ ID NO: 7, SEQ ID NO: 12, or SEQ ID NO: 32.

In the second aspect of the present invention, it provides a fusion protein complex molecule which has a structure from the N-terminus to the C-terminus as shown in Formula Ic or Id:

A2...A1-L-B (Ic);

B-L-A1...A2 (Id);

wherein,
elements A1 and A2 are each independently a polypeptide molecule without an immune effect;
element B is a polypeptide receptor molecule or component thereof, or fragment thereof that specifically binds a pMHC epitope; and
element L is a flexible linker;
"-" is a covalent bond;
"..." is a disulfide bond or non-covalent interaction between protein domains.

In some specific embodiments, the "..." is a disulfide bond.

In some specific embodiments, the elements A1, A2 are each independently selected from the group consisting of: a heavy chain variable region of an antibody, or a light chain variable region of an antibody. For example, the element A1 is a heavy chain variable region of the antibody and the element A2 is a light chain variable region of the antibody; or, the element A1 is a light chain variable region of the antibody and the element A2 is a heavy chain variable region of the antibody. The antibody of the present invention may be UCHT1, OK3, 12F6, etc., or a mutant molecule of these antibodies, wherein the amino acid sequence of the mutant molecule has at least 95% or more, at least 98% or more, at least 99% or more, at least 99.9% or more identity with the amino acid sequence of the corresponding wild-type molecule.

In some specific embodiments, the amino acid sequence of the heavy chain variable region of the antibody comprises a sequence as shown in SEQ ID NO: 14, SEQ ID NO: 20, or SEQ ID NO: 5.

In some specific embodiments, the amino acid sequence of the light chain variable region of the antibody comprises a sequence as shown in SEQ ID NO: 16, SEQ ID NO: 22, or SEQ ID NO: 10.

In some specific embodiments, the amino acid sequence of the heavy chain variable region of the antibody is a sequence as shown in SEQ ID NO: 14, SEQ ID NO: 20 or SEQ ID NO: 5.

In some specific embodiments, the amino acid sequence of the light chain variable region of the antibody is a sequence as shown in SEQ ID NO: 16, SEQ ID NO: 22, or SEQ ID NO: 10.

In some specific embodiments, the element B may comprise a molecule selected from the group consisting of: a TCR molecule, a single chain αβTCR, a TCRα chain, a TCRβ chain, an antibody molecule Fab, a single chain antibody molecule, and combinations thereof.

In some specific embodiments, the amino acid sequence of the TCRα chain is shown in SEQ ID NO: 1.

In some specific embodiments, the amino acid sequence of the TCRβ chain is shown in SEQ ID NO: 3.

In some specific embodiments, the element B is a TCR molecule, the amino acid sequence of the TCRα chain of the TCR molecule is shown in SEQ ID NO: 1, and the amino acid sequence of the TCRβ chain of the TCR molecule is shown in SEQ ID NO: 3.

In some specific embodiments, the element B is a TCR molecule, the amino acid sequence of the TCRα chain of the TCR molecule is shown in SEQ ID NO: 1, the amino acid sequence of the TCRβ chain of the TCR molecule is shown in SEQ ID NO: 3, and the element A1 is coupled to the N-terminus of the TCRβ chain via L.

In some specific embodiments, the element L comprises the structure (GGGGS)n, wherein n is a positive integer from 1 to 5, preferably comprises a sequence as shown in SEQ ID NO: 9.

In the third aspect of the present invention, it provides a polynucleotide encoding the fusion protein of the first aspect of the present invention or the fusion protein complex molecule of the second aspect of the present invention.

In some specific embodiments, the polynucleotide encodes a protein or polypeptide selected from the group consisting of: the fusion protein of the first aspect of the present invention, element A1, element A2, element B (including B1, B2), a single chain αβTCR, a TCRα chain, a TCRβ chain, an antibody molecule Fab, or a single-chain antibody molecule.

In some specific embodiments, the polynucleotide has a nucleotide sequence as shown in SEQ ID NO: 2, 4, 15, 17, 19, 21, 23, 25, 27, 29, 6, 8, 11, 13, 31 or 33.

In some specific embodiments, the polynucleotide has a nucleotide sequence as shown in SEQ ID NO: 19, SEQ ID NO: 25, SEQ ID NO: 27, SEQ ID NO: 29, SEQ ID NO: 8, SEQ ID NO: 13, or SEQ ID NO: 33.

In some specific embodiments, the polynucleotide comprises DNA or RNA.

In the fourth aspect of the present invention, it provides an expression vector comprising the polynucleotide of the third aspect of the present invention.

In some specific embodiments, the expression vector is selected from the group consisting of: DNA, RNA, a viral vector, a plasmid, a transposon, other gene transfer systems, and combinations thereof.

Preferably, the expression vector comprises a viral vector such as a lentivirus, an adenovirus, an AAV virus, a retrovirus, and combinations thereof.

In the fifth aspect of the present invention, it provides a host cell comprising the expression vector of the fourth aspect of the present invention, or having the polynucleotide of the third aspect of the present invention integrated into its genome.

In some specific embodiments, the host cell comprises a prokaryotic or eukaryotic cell.

In some specific embodiments, the host cell is selected from the group consisting of: an *E. coli* cell, a yeast cell, a mammalian cell, a phage, and combinations thereof.

In some specific embodiments, the prokaryotic cell is selected from the group consisting of: *Escherichia coli, Bacillus subtilis,* Lactic acid bacteria, Streptomyces, *Proteus mirabilis,* and combinations thereof.

In some specific embodiments, the eukaryotic cell is selected from the group consisting of: Pichia pastoris, *Saccharomyces cerevisiae,* fission yeast, trichoderma, and combinations thereof.

In some specific embodiments, the eukaryotic cell is selected from the group consisting of: an insect cell such as fall armyworm, a plant cell such as tobacco, a BHK cell, a CHO cell, a COS cell, a myeloma cell, and combinations thereof.

In some specific embodiments, the host cell is preferably a mammalian cell, more preferably a HEK293 cell, a CHO cell, a BHK cell, a NSO cell, or a COS cell.

In some specific embodiments, the host cell is Pichia pastoris.

In the sixth aspect of the present invention, it provides a method of generating the fusion protein of the first aspect of the present invention or the fusion protein complex molecule of the second aspect of the present invention, which comprises the steps of:
(a) culturing the host cell of the fifth aspect of the present invention under conditions suitable for the production of the fusion protein of the first aspect of the present invention or the fusion protein complex molecule of the second aspect of the present invention, thereby obtaining a culture containing the fusion protein or the fusion protein complex molecule;
(b) isolating or recovering the fusion protein or fusion protein complex molecule from the culture; and
(c) optionally, purifying and/or modifying the fusion protein or fusion protein complex molecule obtained in step (b).

In some specific embodiments, the fusion protein has an amino acid sequence as shown in SEQ ID NO: 18, SEQ ID NO: 24, SEQ ID NO: 26, SEQ ID NO: 28, SEQ ID NO: 7, SEQ ID NO: 12 or SEQ ID NO: 32.

In the seventh aspect of the present invention, it provides an immunoconjugate comprising:
(a) the fusion protein of the first aspect of the present invention or the fusion protein complex molecule of the second aspect of the present invention; and
(b) a coupling moiety selected from the group consisting of: detectable marker, drug, toxin, cytokine, radionuclide, enzyme, gold nanoparticle/nanorod, magnetic nanoparticle, viral capsid protein or VLP, and combinations thereof.

In some specific embodiments, the radionuclide include:
(i) a diagnostic isotope, wherein the diagnostic isotope is selected from the group consisting of: Tc-99m, Ga-68, F-18, 1-123, 1-125, 1-131, In-111, Ga-67, Cu-64, Zr-89, C-11, Lu-177, Re-188, and combinations thereof; and/or
(ii) a therapeutic isotope, wherein the therapeutic isotope is selected from the group consisting of: Lu-177, Y-90, Ac-225, As-211, Bi-212, Bi-213, Cs-137, Cr-51, Co-60, Dy-165, Er-169, Fm-255, Au-198, Ho-166, 1-125, 1-131 Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133, Yb-169, Yb-177, and combinations thereof.

In some specific embodiments, the coupling moiety is a drug or toxin.

In some specific embodiments, the drug is a cytotoxic drug.

In some specific embodiments, the cytotoxic drug is selected from the group consisting of: an antitubulin drug, a DNA minor groove binding reagent, a DNA replication inhibitor, an alkylation reagent, an antibiotic, a folate antagonist, an antimetabolite drug, a chemotherapeutic sensitizing agent, a topoisomerase inhibitor, vinca alkaloids, and combinations thereof.

In some specific embodiments, examples of particularly useful cytotoxic drugs include, for example, DNA minor groove binding reagents, DNA alkylation reagents, and tubulin inhibitors; and typical cytotoxic drugs include, for example, auristatins, camptothecins, duocarmycins, etoposides, maytansines, and maytansinoids (e.g. DM1 and DM4), taxanes, benzodiazepines, or benzodiazepine containing drugs (e.g., pyrrolo[1,4]benzodiazepines (PBDs), indolinobenzodiazepines and oxazolidinobenzodiazepines), vinca alkaloids, and combinations thereof.

In some specific embodiments, the toxin is selected from the group consisting of: auristatins (e.g., auristatin E, auristatin F, MMAE, and MMAF), chlortetracycline, mimetasinol, ricin, ricin A-chain, combretastatin, duokamixin, dolastatin, adriamycin, daunorubicin, paclitaxel, cisplatin, cc1065, ethidium bromide, mitomycin, etoposide, tenoposide, vincristine, vinblastine, colchicine, dihydroxyanthraquinone, actinomycin, diphtheria toxin, pseudomonas exotoxin (PE) A, PE40, jequiritin, j equiritin A-chain, modeccin A-chain, α-sarcina, gelonin, mitogellin, retstrictocin, phenomycin, enomycin, curicin, crotin, calicheamicin, Sapaonaria officinalis inhibitor, glucocorticoid, and combinations thereof.

In some specific embodiments, the coupling moiety is a detectable marker.

In some specific embodiments, the coupling moiety is selected from the group consisting of: fluorescent or luminescent markers, radioactive labels, MRI (Magnetic Resonance Imaging) or CT (Computerized Tomography) contrast agents, or enzymes capable of producing detectable products, radionuclides, biotoxins, cytokines (e.g., IL-2, etc.), antibodies, antibody Fc fragments, antibody scFv fragments, gold nanoparticles/nanorods, virus particles, liposomes, magnetic nanoparticles, prodrug activators (e.g., DT-diaphorase (DTD) or biphenyl hydrolase-like (BPHL) proteins), and nanoparticles in any forms.

In the eighth aspect of the present invention, it provides a pharmaceutical composition comprising:
(i) the fusion protein of the first aspect of the present invention, the fusion protein complex molecule of the second aspect of the present invention, the polynucleotide of the third aspect of the present invention, the expression vector of the fourth aspect of the present invention, the host cell of the fifth aspect of the present invention, or the immunoconjugate of the seventh aspect of the present invention, and combinations thereof;
(ii) optionally, element A2; and
(iii) a pharmaceutically acceptable carrier.

In some specific embodiments, the element A2 is selected from the group consisting of: a heavy chain variable region of an antibody, a light chain variable region of an antibody, and combinations thereof.

In some specific embodiments, the coupling moiety of the immunoconjugate is a drug, a toxin, and/or a therapeutic isotope.

In some specific embodiments, the pharmaceutical composition is in injectable form.

In some specific embodiments, the pharmaceutical composition also contains other drugs for treating tumors.

In some specific embodiments, the pharmaceutical composition is used in the manufacture of a medicament for preventing and/or treating a tumor.

In the ninth aspect of the present invention, it provides a method of treating a disease, which comprises the step of: administering the fusion protein of the first aspect of the present invention, the fusion protein complex molecule of the second aspect of the present invention, the immunoconjugate of the seventh aspect of the present invention, the pharmaceutical composition of the eighth aspect of the present invention, to a subject in need thereof.

In some specific embodiments, the subject includes mammals, such as humans, marmosets.

In the tenth aspect of the present invention, it provides a recombinant protein comprising:
(i) the fusion protein of the first aspect of the present invention, or the fusion protein complex molecule of the second aspect of the present invention; and
(ii) an optional tag sequence to assist in expression and/or purification.

In some specific embodiments, the tag sequence includes Fc tags, HA tags, and 6His tags.

In the eleventh aspect of the present invention, it provides use of the fusion protein of the first aspect of the present invention, the fusion protein complex molecule of the second aspect of the present invention, the polynucleotide of third aspect of the present invention, the expression vector of the fourth aspect of the present invention, the host cell of the fifth aspect of the present invention, the immunoconjugate of the seventh aspect of the present invention, the pharmaceutical composition of the eighth aspect of the present inventio, or the recombinant protein of the tenth aspect of the present invention, in the manufacture of a medicament for preventing and/or treating a tumor in a subject.

In some specific embodiments, the tumor includes but is not limited to: breast cancer, lung cancer, liver cancer, and combinations thereof.

In some specific embodiments, the subject is a mammal, preferably a human.

It should be understood that within the scope of the present invention, the above-mentioned technical features of the present invention and the technical features specifically described in the following (such as the examples) can be combined with each other to form a new or preferred technical solution, which are not redundantly repeated one by one due to space limitation.

### Description of the drawings

Figure 1a shows the amino acid sequence of the 1G4 TCR α chain (SEQ ID No: 1).
Figure 1b shows the nucleic acid sequence of the 1G4 TCR α chain (SEQ ID No: 2).
Figure 2a shows the amino acid sequence of the 1G4 TCR β chain (SEQ ID No: 3).
Figure 2b shows the nucleic acid sequence of the 1G4 TCR β chain (SEQ ID No: 4).
Figure 3a shows the amino acid sequence of the heavy chain variable region of the antibody (SEQ ID No: 5).
Figure 3b shows the nucleic acid sequence of the heavy chain variable region of the antibody (SEQ ID No: 6).
Figure 4a shows the amino acid sequence obtained by fusing the N-terminus of the 1G4 TCR β chain with the heavy chain variable region of the antibody (SEQ ID No: 7).
Figure 4b shows the nucleic acid sequence obtained by fusing the N-terminus of the 1G4 TCR β chain with the heavy chain variable region of the antibody (SEQ ID No: 8).
Figure 5 shows the amino acid sequence of the peptide linker (SEQ ID No: 9).
Figure 6a shows the amino acid sequence of the light chain variable region of the antibody (SEQ ID No: 10).
Figure 6b shows the nucleic acid sequence of the light chain variable region of the antibody (SEQ ID No: 11).
Figure 7a shows the amino acid sequence obtained by fusing the N-terminus of the 1G4 TCR β chain with the light chain variable region of the antibody (SEQ ID No: 12).
Figure 7b shows the nucleic acid sequence obtained by fusing the N-terminus of the 1G4 TCR β chain with the light chain variable region of the antibody (SEQ ID No: 13).
Figure 8a shows the sequence obtained by substitution of Gly44Cys in the amino acid sequence of the heavy chain variable region of the antibody (SEQ ID No: 14).
Figure 8b shows the nucleic acid sequence (SEQ ID No: 15) encoding the sequence shown in Figure 8a (i.e., SEQ ID No: 14).
Figure 9a shows the sequence obtained by substitution of Gln100Cys in the amino acid sequence of the light chain variable region of the antibody (SEQ ID No: 16).
Figure 9b shows the nucleic acid sequence (SEQ ID No: 17) encoding the sequence shown in Figure 9a (i.e., SEQ ID No: 16).
Figure 10a shows the sequence obtained by substitution of Gly44Cys in the amino acid sequence which is obtained by fusing the N-terminus of the 1G4 TCR β chain with the heavy chain variable region of the antibody (SEQ ID No: 18).
Figure 10b shows the nucleic acid sequence (SEQ ID No: 19) encoding the sequence shown in Figure 10a (i.e., SEQ ID No: 18).
Figure 11a shows the sequence obtained by substitution of Gln105Cys in the amino acid sequence of the heavy chain variable region of the antibody (SEQ ID No: 20).
Figure 11b shows the nucleic acid sequence (SEQ ID No: 21) encoding the sequence shown in Figure 11a (i.e., SEQ ID No: 20).
Figure 12a shows the sequence obtained by substitution of Ala43Cys in the amino acid sequence of the light chain variable region of the antibody (SEQ ID No: 22).
Figure 12b shows the nucleic acid sequence (SEQ ID No: 23) encoding the sequence shown in Figure 12a (i.e., SEQ ID No: 22).
Figure 13a shows the sequence obtained by substitution of Gln105Cys in the amino acid sequence which is obtained by fusing the N-terminus of the 1G4 TCR β chain with the heavy chain variable region of the antibody (SEQ ID No: 24).
Figure 13b shows the nucleic acid sequence (SEQ ID No: 25) encoding the sequence shown in Figure 13a (i.e., SEQ ID No: 24).
Figure 14a shows the sequence obtained by substitution of Ala43Cys in the amino acid sequence which is obtained by fusing the N-terminus of the 1G4 TCR β chain with the light chain variable region of the antibody (SEQ ID No: 26).
Figure 14b shows the nucleic acid sequence (SEQ ID No: 27) encoding the sequence shown in Figure 14a (i.e., SEQ ID No: 26).
Figure 15a shows the sequence obtained by substitution of Gln100Cys in the amino acid sequence which is obtained by fusing the N-terminus of the 1G4 TCR β chain with the light chain variable region of the antibody (SEQ ID No: 28).
Figure 15b shows the nucleic acid sequence (SEQ ID No: 29) encoding the sequence shown in Figure 15a (i.e., SEQ ID No: 28).
Figure 16a shows the amino acid sequence of the AFP TCR α chain (SEQ ID No: 30).
Figure 16b shows the nucleic acid sequence of the AFP TCR α chain (SEQ ID No: 31).
Figure 17a shows the sequence obtained by substitution of Gln105Cys in the amino acid sequence which is obtained by fusing the N-terminus of the AFP TCR β chain with the heavy chain variable region of the antibody (SEQ ID No: 32).
Figure 17b shows the nucleic acid sequence (SEQ ID No: 33) encoding the sequence shown in Figure 17a (i.e., SEQ ID No: 32).
Figure 18 shows the amino acid sequence of TRAC*01 (SEQ ID No: 34).
Figure 19 shows the amino acid sequence of TRBC*01 (SEQ ID No: 35).
Figure 20 shows the amino acid sequence of TRBC*02 (SEQ ID No: 36).
Figure 21 shows the 1G4 TCR binding epitope NY-ESO-1 (157-165) (SEQ ID No: 37).
Figure 22 shows the AFP TCR binding epitope AFP (158-166) (SEQ ID No: 38).
Figure 23 shows a schematic diagram of the VH-TCRβ form of the double-domain fusion protein.
Figure 24 shows a schematic diagram of the VL-TCRβ form of the double-domain fusion protein.
Figure 25 shows a schematic diagram of the VH44-TCRβ form of the triple-domain fusion molecule.
Figure 26 shows a schematic diagram of the VH105-TCRβ form of the triple-domain fusion molecule.
Figure 27 shows a schematic diagram of the VL43-TCRβ form of the triple-domain fusion molecule.
Figure 28 shows a schematic diagram of the VL100-TCRβ form of the triple-domain fusion molecule.
Figure 29 shows an SDS-PAGE plot of the double-domain fusion protein in the form of VH-TCRβ and VL-TCRβ.
Figure 30 shows an SDS-PAGE plot of the triple-domain fusion molecule in the form of VH44-TCRβ and VH105-TCRβ.
Figure 31 shows an SDS-PAGE plot of the triple-domain fusion molecule in the form of VL43-TCRβ and VL100-TCRβ.
Figure 32 shows an SDS-PAGE plot of the AFP VH105-TCRβ form of the triple-domain fusion molecule.
Figure 33a shows the LDH assay detecting the 1G4 double-domain fusion molecule (VH-TCRβ, VL-TCRβ forms) mediated CD8+ cell killing on MDA-MB-231 (A2-positive, NY-ESO-1-negative, breast cancer cell line).
Figure 33b shows the LDH assay detecting the 1G4 double-domain fusion molecule (VH-TCRβ, VL-TCRβ forms) mediated CD8+ cell killing on MDA-MB-231 (A2-positive, NY-ESO-1-positive, breast cancer cell line).
Figure 34a shows the LDH assay detecting the 1G4 double-domain fusion molecule (VH-TCRβ, VL-TCRβ forms) mediated CD8+ cell killing on NCI-H1299-A2 (NY-ESO-1-positive, A2-negative, lung cancer cell line).
Figure 34b shows the LDH assay detecting the 1G4 double-domain fusion molecule (VH-TCRβ, VL-TCRβ forms) mediated CD8+ cell killing on NCI-H1299-A2 (NY-ESO-1 positive, A2-positive, lung cancer cell line).
Figure 35a shows the LDH assay detecting the 1G4 multi-domain fusion molecule (VH44-TCRβ, VH105-TCRβ, VL43-TCRβ, VL100-TCRβ forms) mediated CD8+ cell killing on NCI-H1299-A2 (NY-ESO-1-positive, A2-positive, lung cancer cell line).
Figure 35b shows the LDH assay detecting the 1G4 multi-domain fusion molecule (VH44-TCRβ, VH105-TCRβ, VL43-TCRβ, VL100-TCRβ forms) mediated CD8+ cell killing on NCI-H1299 (NY-ESO-1-positive, A2-negative, lung cancer cell line).
Figure 36a shows the LDH assay detecting the 1G4 multi-domain fusion molecule (VH44-TCRβ, VH105-TCRβ) mediated CD8+ cell killing on MDA-MB-231 (A2-positive, NY-ESO-1-positive, breast cancer cell line).
Figure 36b shows the LDH assay detecting the 1G4 multi-domain fusion molecule (VH44-TCRβ, VH105-TCRβ) mediated CD8+ cell killing on MDA-MB-231 (A2-positive, NY-ESO-1-negative, breast cancer cell line).
Figure 37 shows the LDH assay detecting the AFP multi-domain fusion molecule (VH105-TCRβ form) mediated CD8+ cell killing on HepG2 (A2-positive, AFP-positive, liver cancer cell line) and NCI-H1299-A2 (A2-positive, AFP-negative, lung cancer cell line).

### Detailed Description

After extensive and intensive research and massive screening, the inventors have unexpectedly discovered a class of fusion proteins (e.g. fusion proteins with a structure from the N-terminus to the C-terminus as shown in Formula Ia or Ib) for the first time, which are formed by the coupling of an antibody single chain molecule A1 without an immune effect, and a polypeptide receptor molecule or component thereof, or a fragment thereof, B, that specifically binds pMHC epitopes, via L. The fusion proteins can form a fusion protein complex molecule with dual biological functions (e.g. a fusion protein complex molecule with a structure from the N-terminus to the C-terminus as shown in Formula Ic or Id) after simple mixing with element A2 (preferably an element A2 without an immune effect). The fusion protein complex molecule is a multi-domain fusion molecule close to its natural state, requiring no peptide segment or only requiring a shorter peptide segment (e.g. 5 amino acids GGGGS) to connect element A1 (preferably an element A1 without an immune effect) and element B that specifically binds the pMHC antigen. This can avoid the risk of immunogenicity that may exist in conventional fusion proteins due to the introduction of an artificial flexible peptide chain (usually greater than 5 amino acids) to link the various domains into a single polypeptide molecule. The fusion protein complex molecule of the present invention is easy to generate and close to the natural state. On this basis, the inventors have completed the present invention.

Specifically, in the present invention, the inventors coupled the mutated antibody heavy chain variable region VH without an immune effect or the antibody light chain variable region VL without an immune effect, to the N-terminus of the TCRβ chain by GGGGS, and then mixed the conjugate with the TCRα chain to form a fusion protein under suitable conditions, but this fusion protein cannot induce T cell activation. However, when the above molecule is mixed with VL, the light chain variable region of the antibody without an immune effect, or VH, the heavy chain variable region of the antibody without an immune effect, another fusion protein complex molecule can be obtained by reduction, denaturation and dialysis. The experimental results show that the fusion protein complex molecule has the ability to redirect CD8⁺ T cells to kill a variety of tumor cells (e.g., the MDA-MB-231 breast cancer cell line, the NCI-H1299 lung cancer cell line, and the HepG2 liver cancer cell line, etc.).

### Terms

Before describing the present invention, it should be understood that the present invention is not limited to the described specific methods and experimental conditions, as such methods and conditions may vary. It should also be understood that the terms used herein are intended only to describe specific implementation schemes, and their intent is not restrictive. The scope of the present invention will be limited only by the accompanying claims.

Unless otherwise defined, all technical and scientific terms used herein have the same meanings as those commonly understood by those skilled in the art to which the present invention belongs.

Although any method and material similar or equivalent to the present invention may be used in the implementation or testing of the present invention, preferred methods and materials are exemplified herein.

### Antibody

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of light chain pairs with the first constant region of heavy chain, and the variable region of light chain pairs with the variable region of heavy chain. Special amino acid residues form an interface between the variable regions of a light chain and a heavy chain.

As used herein, the term "variable" means that certain portion of the variable region in an antibody differs in sequences, which is responsible for the binding and specificity of various specific antibodies to their specific antigens. However, the variability is not distributed evenly throughout the entire variable regions of an antibody. It is concentrated in three fragments called complementarity determining regions (CDRs) or hypervariable regions in light chain and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partial β-sheet structure. The CDRs in each chain are closely linked together via the FR regions, and form the antigen binding site of an antibody together with the CDRs of the other chain (see Kabat et al., NIH Publ. No. 91-3242, Volume I, pages 647-669 (1991)). Constant regions are not directly involved in the binding of antibodies to antigen, however, they exhibit different effector functions, such as participating in the antibody-dependent cytotoxicity of antibodies.

As known to those skilled in the art, immunoconjugates and fusion expression products include: conjugates formed by the combination of drugs, toxins, cytokines, radionuclides, enzymes, and other diagnostic or therapeutic molecules with the antibody or fragment thereof of the present invention.

As used herein, the terms "heavy chain variable region" and "VH" can be used interchangeably; and the terms "light chain variable region" and "VL" can be used interchangeably.

As used herein, the terms "variable region" and "complementarity determining region (CDR)" can be used interchangeably.

In a preferred embodiment of the present invention, the heavy chain variable region of the antibody comprises three complementarity determining regions, CDR1, CDR2, and CDR3.

In general, the antigen binding characteristics of an antibody can be described by three specific regions located in the heavy chain variable region, called the variable regions (CDRs), which are separated by four frame regions (FRs). The amino acid sequences of the four FRs are relatively conservative and do not directly participate in the binding reaction. These CDRs form a loop structure, and the β-sheets formed by the FRs in between are spatially close to each other, and the CDRs on the heavy chain and the CDRs on the corresponding light chain constitute the antigen-binding site of the antibody. It can be determined which amino acids constitute the FR or CDR region by comparing the amino acid sequences of antibodies of the same type.

The variable regions of the heavy chains of the antibody of the present invention are of particular interest because at least part of them involve binding antigens. Therefore, the present invention includes those molecules with an antibody heavy chain variable region containing CDRs, as long as their CDRs have more than 90% (preferably more than 95%, most preferably more than 98%) homology with the CDRs identified here.

The present invention includes not only intact antibodies, but also fragments of immunologically active antibodies or fusion proteins formed by antibodies with other sequences. Thus, the present invention also includes fragments, derivatives and analogs of the antibody.

As used herein, the terms "fragment", "derivative" and "analog" refer to a polypeptide that substantially retain the same biological function or activity of the antibody of the present invention. The polypeptide fragment, derivative or analog of the present invention may be (i) a polypeptide with one or more conservative or non-conservative amino acid residues (preferably conservative amino acid residues) substituted, and such substituted amino acid residues may or may not be encoded by the genetic code, or (ii) a polypeptide with a substituent group in one or more amino acid residues, or (iii) a polypeptide formed by fusion of a mature polypeptide with another compound (such as a compound that extends the half-life of the polypeptide, such as polyethylene glycol), or (iv) a polypeptide formed by fusion of an additional amino acid sequence to the polypeptide sequence (such as a leader sequence or secretory sequence, or a sequence or protein sequence used to purify the polypeptide, or a fusion protein formed with a 6His tag). According to the teachings herein, these fragments, derivatives and analogs are within the scope of knowledge of those skilled in the art.

The variant forms of the polypeptide include homologous sequences, conservative variants, alleles, natural mutants, induced mutants, proteins encoded by DNA capable of hybridizing with the coding DNA of the antibody of the present invention under high or low tightness conditions, and polypeptides or proteins obtained by using anti-serum against the antibody of the present invention.

In the present invention, "conservative variant of the antibody of the present invention" refers to a polypeptide formed by replacing at most 10, preferably at most 8, more preferably at most 5, and most preferably at most 3 amino acids with amino acids of close or similar properties as compared with the amino acid sequence of the antibody of the present invention. These conservative variant polypeptides are best produced by amino acid substitution according to Table 1.

**Table 1**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

Typically, in the present invention, a stable dimer is obtained by artificially introducing cysteines into the heavy chain variable region and light chain variable region of the antibody without an immune effect, forming an inter-chain disulfide bond.

Specifically, in a preferred embodiment of the present invention, a disulfide bond connection between heavy and light chains is established by replacing the heavy chain variable region Gly44 and the light chain variable region GlnlOO with cysteines. In another preferred embodiment of the present invention, a disulfide bond connection between heavy and light chains is established by replacing the heavy chain variable region Ala43 and the light chain variable region Gln105 with cysteines. In this way, there is no need to introduce flexible peptides with 15-30 amino acids between the heavy and light chains of the antibody, avoiding the formation of non-human molecules or non-human protein surface antigen determinants that can induce immune responses, and thereby reducing the immunogenicity risk of this bifunctional molecule.

### T cell receptor, TCR

The international ImMunoGeneTics information system (IMGT) can be used to describe TCR. Natural αβ heterodimeric TCR has α chain and β chain. Broadly speaking, each chain includes a variable region, a linker region, and a constant region, and β chains usually contain a short hypervariable region between the variable region and the linker region, but this hypervariable region is often considered as part of the linker region. The linker region of TCR is determined through unique IMGT's TRAJ and TRBJ, and the constant region of TCR is determined through IMGT's TRAC and TRBC.

Each variable region contains three CDRs (complementarity determining regions), CDR1, CDR2, and CDR3, embedded in the framework sequences. In the IMGT naming convention, the different numbers of TRAV and TRBV refer to different Vα type and Vβ type. In the IMGT system, the α chain constant region has the following symbol: TRAC*01, wherein "TR" represents the T cell receptor gene; "A" represents α chain gene; C represents the constant region; "*01" represents allele 1. The β chain constant domain has the following symbols: TRBC1*01 or TRBC2*01, wherein "TR" represents the T cell receptor gene; "B" represents β chain gene; C represents the constant region; "*01" represents allele 1. The constant region of the α chain is uniquely determined, and in the form of β chains, there are two possible constant region genes "C1" and "C2". Those skilled in this field can obtain the constant region gene sequence of the TCR α and β chains through publicly available IMGT databases.

TCR's α and β chains are generally regarded as having two "domains" respectively, i.e., variable domains and constant domains. Variable domains are composed of connected variable regions and linker regions. Therefore, in the specification and claims of the present application, "TCR α chain variable domain" refers to the connected TRAV and TRAJ regions, similarly, "TCR β chain variable domain" refers to the connected TRBV and TRBD/TRBJ regions. The three CDRs of the TCR α chain variable domain are CDR1α, CDR2α and CDR3α, respectively; and the three CDRs of the TCR β chain variable domain are CDR1β, CDR2β and CDR3β, respectively. The framework sequence of the TCR variable domain of the present invention can be mouse-derived or human-derived, preferably human-derived.

The constant domain of TCR includes the intracellular portion, transmembrane region, and extracellular portion.

In a preferred embodiment of the present invention, the TCR portion is a heterodimer, wherein α and β polypeptides include the variable and constant regions of TCR molecules, but do not have intracellular and transmembrane regions. The heterodimer is connected by artificially introduced inter-chain disulfide bonds. Specifically, the constant region of the α and β polypeptides is linked by a disulfide bond between Thr47 in exon 1 of TRAC*01 and Ser56 in exon 1 of TRBC*01.

Preferably, the TCR molecules of the present invention are heterodimeric αβTCP polypeptide pairs, wherein the α and β polypeptides each contain TCR variable and constant regions, but do not have TCR transmembrane and intracellular regions.

More preferably, the amino acid sequence of the TCR of the present invention refers to the extracellular amino acid sequence of the TCR.

In a preferred embodiment of the present invention, the constant region of α and β polypeptides of the TCR molecule is connected by a disulfide bond formed by replacing the 47th threonine (Thr) in exon 1 of TRAC*01 and the 56th serine (Ser) in exon 1 of TRBC*01 or TRBC*02 with cysteines (Cys).

In a preferred embodiment of the present invention, the constant region of α and β polypeptides of the TCR molecule is connected by a disulfide bond formed by replacing the 52nd arginine (Arg) in exon 1 of TRAC*01 and the 53rd serine (Ser) in exon 1 of TRBC*01 or TRBC*02 of the IMTG numbering method with cysteines (Cys).

In a preferred embodiment of the present invention, the constant region of α and β polypeptides of the TCR molecule is connected by a disulfide bond formed by replacing the 88th proline (Pro) in exon 1 of TRAC*01 and the 18th alanine (Ala) in exon 1 of TRBC*01 or TRBC*02 of the IMTG numbering method with cysteines (Cys).

In a preferred embodiment of the present invention, the constant region of α and β polypeptides of the TCR molecule is connected by a disulfide bond formed by replacing the 9th tyrosine (Tyr) in exon 1 of TRAC*01 and the 19th glutamic acid (Glu) in exon 1 of TRBC*01 or TRBC*02 with cysteines (Cys).

In a preferred embodiment of the present invention, the amino acid sequence of the TCR α chain is shown in SEQ ID NO: 1.

In a preferred embodiment of the present invention, the amino acid sequence of the TCR β chain is shown in SEQ ID NO: 3.

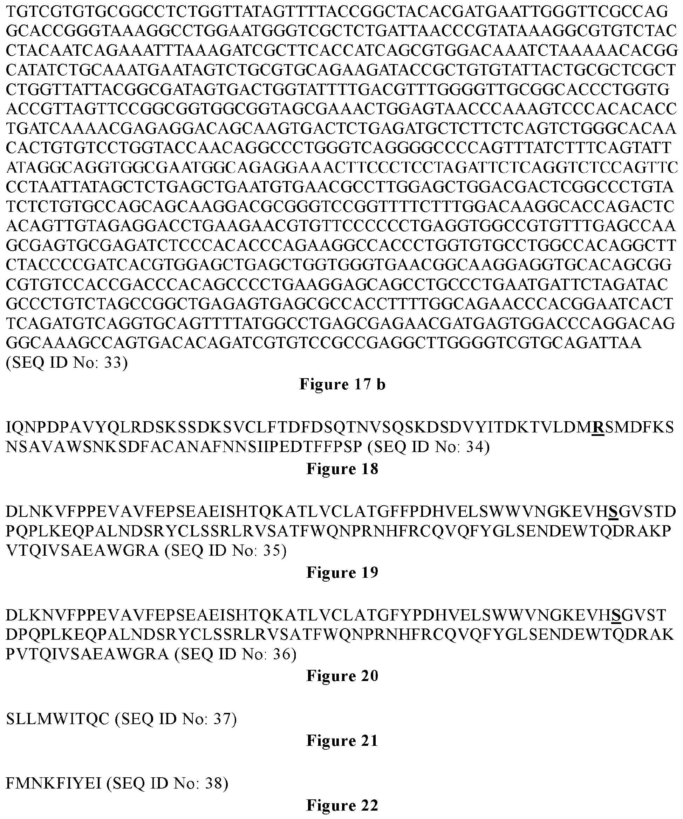

In a preferred embodiment of the present invention, the amino acid sequence of TRAC*01 is shown in SEQ ID NO: 34 (see Figure 18).

In a preferred embodiment of the present invention, the amino acid sequence of TRBC*01 is shown as SEQ ID NO: 35 (see Figure 19).

In a preferred embodiment of the present invention, the amino acid sequence of TRBC*02 is shown in SEQ ID NO: 36 (see Figure 20).

### Fusion protein

As used herein, fusion protein refers to a single fused protein polypeptide chain in which the genetic code of the protein molecule is contiguous and consists of two or more domains translated by a single mRNA and refers to a fusion between domains of a non-natural state.

In the present invention, it provides a fusion protein, the structure of which from the N-terminus to the C-terminus is as shown in Formula Ia or Ib:

A1-L-B (Ia);

B-L-A1 (Ib);

wherein,
element A1 is a polypeptide molecule without an immune effect;
element B is a polypeptide receptor molecule or component thereof, or fragment thereof that specifically binds a pMHC epitope;
element L is a flexible linker;
"-" is a covalent bond.

Preferably, the structure of the fusion protein from the N-terminus to the C-terminus is as shown in Formula Ia.

Preferably, in the fusion protein, the N-terminus of B (a polypeptide receptor molecule or component thereof, or fragment thereof that specifically binds a pMHC epitope) is connected to the C-terminus of A1 (an antibody single-chain molecule without an immune effect).

Preferably, the flexible linker is a peptide linker.

Preferably, the peptide linker is GGGGS (SEQ ID No: 9).

Preferably, the element A1 is selected from a heavy chain variable region of an antibody or a light chain variable region of an antibody.

In another preferred embodiment of the present invention, in the antibody, the glycine (Gly) at position 44 of the heavy chain variable region of the antibody (Kabat numbering system, hereinafter the same) is substituted with cysteine (Cys); and the glutamine (Gln) at position 100 of the light chain variable region of the antibody is substituted with cysteine (Cys).

In a preferred embodiment of the present invention, in the antibody, the glutamine (Gln) at position 105 of the heavy chain variable region of the antibody is substituted with cysteine (Cys); and the alanine (Ala) at position 43 of the light chain variable region of the antibody is substituted with cysteine (Cys)

In a preferred embodiment of the present invention, the element A1 binds to a CD3 antigen.

In a preferred embodiment of the present invention, the antibody is selected from the group consisting of: UCHT1, OKT3, or 12F6.

In a preferred embodiment of the present invention, the amino acid sequence of the heavy chain variable region of the antibody is as shown in SEQ ID NO: 14, SEQ ID NO: 20 or SEQ ID NO: 5.

In a preferred embodiment of the present invention, the amino acid sequence of the light chain variable region of the antibody is as shown in SEQ ID NO: 16, SEQ ID NO: 22 or SEQ ID NO: 10.

Typically, the element B is selected from the group consisting of: a TCR molecule, a single chain αβTCR, a TCRα chain, a TCRβ chain, an antibody molecule Fab, a single chain antibody molecule, and combinations thereof.

In a preferred embodiment of the present invention, the element B is composed of a combination of B1 and B2 via a disulfide bond, wherein B1 is a TCRα chain and B2 is a TCRβ chain; or B1 is a TCRβ chain and B2 is a TCRα chain.

Typically, in the present invention, the N-terminus of the TCR β chain may be connected to a heavy or light chain variable region domain of the antibody that has no immune effect.

In one embodiment of the present invention, the N-terminus of the TCR β chain is connected to the C-terminus of the antibody heavy chain (heavy chain variable region) via a 5-peptide linker GGGGS (SEQ ID No: 9).

In one embodiment of the present invention, the N-terminus of the TCR β chain is connected to the C-terminus of the antibody light chain (light chain variable region) via a 5-peptide linker GGGGS (SEQ ID No: 9).

In a preferred embodiment of the present invention, the N-terminus of the α or β chain of the heterodimer, or the N-terminus of the single chain αβTCR, or the N-terminus of the TCRα chain, or the N-terminus of the TCRβ chain, or the N-terminus of the heavy or light chain of the antibody molecule Fab, or the N-terminus of the single chain antibody molecule, is linked to the C-terminal amino acid of an antibody single-chain molecule without an immune effect.

In a preferred embodiment of the present invention, the element B (i.e., a polypeptide receptor molecule or component thereof, or fragment thereof that specifically binds a pMHC epitope) of the fusion protein specifically binds pMHC, and the element A1 (i.e., an antibody single-chain molecule without an immune effect) is unable to bind antigen molecules of the T cells, or the element A1 can bind antigen molecules of the T cells but is unable to efficiently induce activation of the T cells or produce a physiological effect.

In a preferred embodiment of the present invention, when the fusion protein is mixed with another free polypeptide domain molecule A2 that has no immune effect, the fusion protein and A2 form a fusion protein-A2 complex molecule, and the fusion protein-A2 complex molecule redirects T cells and can effectively activate the redirected T cells and produce an immunophysiological effect.

In a preferred embodiment of the present invention, the TCR molecules are heterodimer αβTCR polypeptide pairs, wherein the α and β polypeptides each contain TCR variable and constant regions, but do not contain TCR transmembrane and intracellular regions.

In a preferred embodiment of the present invention, the constant regions of the α and β polypeptides of the TCR molecules are connected by a disulfide bond formed by replacing the threonine (Thr) at position 47 in exon 1 of TRAC*01 and the serine (Ser) at position 56 in exon 1 of TRBC*01 or TRBC*02 with a cysteine (Cys).

In a preferred embodiment of the present invention, the constant regions of the α and β polypeptides of the TCR molecules are connected by a disulfide bond formed by replacing the arginine (Arg) at position 52 of the IMTG numbering method in exon 1 of TRAC*01 and the serine (Ser) at position 53 in exon 1 of TRBC*01 or TRBC*02 with a cysteine (Cys).

In a preferred embodiment of the present invention, the constant regions of the α and β polypeptides of the TCR molecules are connected by a disulfide bond formed by replacing the proline (Pro) at position 88 of the IMTG numbering method in exon 1 of TRAC*01 and the alanine (Ala) at position 18 in exon 1 of TRBC*01 or TRBC*02 with a cysteine (Cys).

In a preferred embodiment of the present invention, the constant regions of the α and β polypeptides of the TCR molecules are connected by a disulfide bond formed by replacing the tyrosine (Tyr) at position 9 of the IMTG numbering method in exon 1 of TRAC*01 and the glutamic acid (Glu) at position 19 in exon 1 of TRBC*01 or TRBC*02 with a cysteine (Cys).

In a preferred example of the present invention, the amino acid sequence of the TRAC*01 is shown in SEQ ID NO: 34.

In a preferred example of the present invention, the amino acid sequence of the TRBC*01 is shown in SEQ ID NO: 35.

In a preferred example of the present invention, the amino acid sequence of the TRBC*02 is shown in SEQ ID NO: 36.

In a preferred embodiment of the present invention, the amino acid sequence of the TCRα chain is shown in SEQ ID NO: 1.

In a preferred embodiment of the present invention, the amino acid sequence of the TCRβ chain is shown in SEQ ID NO: 3.

In another preferred embodiment of the present invention, the amino acid sequence of the fusion protein is as shown in SEQ ID NO: 18, SEQ ID NO: 24, SEQ ID NO: 26, SEQ ID NO: 28, SEQ ID NO: 7, SEQ ID NO: 12, or SEQ ID NO: 32.

### Fusion protein complex molecule

As used herein, a fusion protein complex molecule is a complex molecule having a single or two or more physiological or biophysical functions, encoded only by two or more genes, respectively, and having two or more protein polypeptide chains translated by two or more mRNAs assembled together extracellularly by non-covalent and/or covalent forms, and refers to polypeptide complex molecules in their unnatural state.

As used herein, the terms "fusion protein complex molecule", "multi-domain fusion molecule close to a natural state", "multi-domain fusion molecule", "fusion protein-A2 complex" have the same meaning and are used interchangeably to refer to a fusion protein complex molecule with dual biological functions (e.g., a fusion protein complex molecule with a structure from the N-terminus to the C-terminus as shown in Formula Ic or Id) formed by the combination of the two elements A1, A2 and L, B. Preferably, the elements A1, A2 have no immune effect.

Preferably, the fusion protein complex molecule of the present invention is obtained by assembly of fusion protein A1-L-B (Ia) or B-L-A1 (Ib) with element A2.

Preferably, the structure of the fusion protein from the N-terminus to the C-terminus is shown in Formula Ic or Id:

A2... A1-L-B (Ic);

B-L-A1...A2 (Id);

wherein,
elements A1 and A2 are each independently an antibody single-chain molecule without an immune effect;
element B is a polypeptide receptor molecule or component thereof, or fragment thereof that specifically binds a pMHC epitope; and
element L is a flexible linker;
"-" is a covalent bond;
"..." is a disulfide bond or non-covalent interaction between protein domains, preferably a disulfide bond.

Preferably, the elements A1, A2 are each independently selected from the group consisting of: a heavy chain variable region of an antibody, or a light chain variable region of an antibody. More preferably, the elements A1, A2 are each independently selected from the group consisting of: a heavy chain variable region of an antibody without an immune effect, or a light chain variable region of an antibody without an immune effect.

In a preferred embodiment of the present invention, the amino acid sequence of the heavy chain variable region of the antibody is as shown in SEQ ID NO: 14, SEQ ID NO: 20 or SEQ ID NO: 5.

In a preferred embodiment of the present invention, the amino acid sequence of the light chain variable region of the antibody is as shown in SEQ ID NO: 16, SEQ ID NO: 22 or SEQ ID NO: 10.

Typically, the element B is selected from the group consisting of: a TCR molecule, a single chain αβTCR, a TCRα chain, a TCRβ chain, an antibody molecule Fab, a single chain antibody molecule, and combinations thereof.

Typically, the amino acid sequence of the TCRα chain is shown in SEQ ID NO: 1.

Typically, the amino acid sequence of the TCRβ chain is shown in SEQ ID NO: 3.

Typically, the element B is a TCR molecule, the amino acid sequence of the TCRα chain of the TCR molecule is shown in SEQ ID NO: 1, and the amino acid sequence of the TCRβ chain of the TCR molecule is shown in SEQ ID NO: 3.

Typically, the element B is composed of a combination of B1 and B2 via a disulfide bond, wherein B1 is a TCRα chain and B2 is a TCRβ chain; or B1 is a TCRβ chain and B2 is a TCRα chain.

In a preferred embodiment of the present invention, the amino acid sequence of the fusion protein is as shown in SEQ ID NO: 18, SEQ ID NO: 24, SEQ ID NO: 26, SEQ ID NO: 28, SEQ ID NO: 7, SEQ ID NO: 12 or SEQ ID NO: 32.

In another preferred embodiment of the present invention, the amino acid sequence of the TCRα chain is shown in SEQ ID NO: 1, and the amino acid sequence of the TCRβ chain is shown in SEQ ID NO: 3.

In another preferred embodiment of the present invention, the element B is a TCR molecule, the amino acid sequence of the TCRα chain of the TCR molecule is shown in SEQ ID NO: 1, the amino acid sequence of the TCRβ chain of the TCR molecule is shown in SEQ ID NO: 3, and the element A1 is coupled to the N-terminus of the TCRβ chain via L.

Typically, the elements A1, A2 are each independently selected from the group consisting of: a heavy chain variable region of an antibody, or a light chain variable region of an antibody. Preferably, the elements A1, A2 are each independently selected from the group consisting of: a heavy chain variable region of an antibody without an immune effect, or a light chain variable region of an antibody without an immune effect.

### Polynucleotide, host cell

The present invention also provides polynucleotide molecules encoding the above-described fusion proteins, element A1, element A2, element B (including B1, B2), single chain αβTCR, TCRα chain, TCRβ chain, antibody molecule Fab, or a single-chain antibody molecule. The polynucleotide of the present invention may be in the form of DNA or in the form of RNA. DNA form includes cDNA, genomic DNA, or synthetic DNA. DNA may be single-stranded or double-stranded. DNA may be coding or non-coding strands.

The polynucleotide encoding the mature polypeptide of the present invention includes: the coding sequence that encodes only the mature polypeptide; the coding sequence of the mature polypeptide and various additional coding sequences; the coding sequence of the mature polypeptide (and optional additional coding sequence) and the non-coding sequence.

The term "polynucleotide encoding a polypeptide" may be a polynucleotide that includes a sequence encoding the polypeptide, or a polynucleotide that also includes additional coding and/or non-coding sequences.

The present invention also relates to a polynucleotide that hybridizes to the above-mentioned sequence and has at least 50%, preferably at least 70%, and more preferably at least 80% identity between the two sequences. In particular, the present invention relates to a polynucleotide that is hybridizable to the polynucleotide of the present invention under strict conditions. In the present invention, "strict conditions" refer to (1) hybridization and elution at lower ionic strength and higher temperature, such as 0.2 × SSC, 0.1% SDS, 60 °C; or (2) hybridization with denaturing agent, such as 50% (v/v) formamide, 0.1% calf serum/0.1% Ficoll, 42 °C, etc.; or (3) hybridization occurs only when the identity between the two sequences is at least 90% or more, more preferably 95% or more. Furthermore, the polypeptide encoded by the hybridizable polynucleotide has the same biological function and activity as the mature polypeptide.

The full-length nucleotide sequence or fragments thereof of the antibody of the present invention may generally be obtained by PCR amplification, recombination or artificial synthesis methods. A feasible method is to synthesize the relevant sequence by artificial synthesis, especially when the fragment length is short. Generally, fragments with a long sequence can be obtained by first synthesizing multiple small fragments and followed by ligation. In addition, the coding sequence of the heavy chain and the expression tag, such as 6His can be fused together to form a fusion protein.

Once the relevant sequence is obtained, the recombination method can be used to obtain the relevant sequence in large quantities. This is usually to clone it into a vector, then transfer it into a cell, and then separate the relevant sequence from the proliferated host cell by conventional methods. The biomolecules (nucleic acids, proteins, etc.) involved in the present invention include biomolecules in isolated form.

At present, the DNA sequence encoding the protein (or its fragment, or its derivative) of the present invention can be obtained completely by chemical synthesis. The DNA sequence can then be introduced into various existing DNA molecules (or, for example, vectors) and cells known in the art. In addition, mutations can be introduced into the protein sequence of the present invention by chemical synthesis.

The present invention also relates to a vector comprising the appropriate DNA sequence as described above and an appropriate promoter or control sequence. These vectors can be used to transform appropriate host cells to express proteins.

Host cells may be prokaryotic cells, such as bacterial cells; or lower eukaryotic cells, such as yeast cells; or higher eukaryotic cells, such as mammalian cells. Representative examples include: *Escherichia coli,* Streptomyces; bacterial cells of *Salmonella typhimurium*; fungal cells such as yeast; insect cells of Drosophila S2 or Sf9; animal cells of CHO, COS7, 293 cells, etc.

Transformation of host cells with recombinant DNA can be carried out using conventional techniques well known to those skilled in the art. When the host is a prokaryotic organism such as *Escherichia coli,* the competent cells capable of absorbing DNA can be harvested after the exponential growth period and treated with CaCl₂, the steps used are well known in the art. Another method is to use MgCl₂. If necessary, the transformation can also be carried out by electroporation. When the host is eukaryotic, the following DNA transfection methods can be used: calcium phosphate co-precipitation method, conventional mechanical methods such as microinjection, electroporation, liposome packaging, etc.

The obtained transformant can be cultured by conventional methods to express the polypeptide encoded by the gene of the present invention. Depending on the host cell used, the medium used in the culture may be selected from a variety of conventional medium. Culture is carried out under conditions suitable for host cell growth. When the host cells grow to an appropriate cell density, the selected promoter is induced by a suitable method (such as temperature conversion or chemical induction), and the cells are cultured for another period of time.

The recombinant polypeptide in the above method may be expressed in the cell, or on the cell membrane, or secreted outside the cell. If necessary, the recombinant protein can be isolated and purified by various separation methods using its physical, chemical and other properties. These methods are well known to those skilled in the art. Examples of these methods include, but are not limited to, conventional renaturation treatment, treatment with a protein precipitant (salting-out method), centrifugation, osmotic shock, ultra-treatment, ultra-centrifugation, molecular sieve chromatography (gel filtration), adsorption chromatography, ion exchange chromatography, high performance liquid chromatography (HPLC) and other liquid chromatography techniques and combinations of these methods.

### Immunoconjugate

The fusion protein and/or the fusion protein complex molecule of the present invention can be used alone, or can be combined or coupled with a detectable label (for diagnostic purposes), a therapeutic agent, a PK (protein kinase) modifying moiety, or any combination of these substances.

A detectable label for diagnostic purposes includes, but is not limited to: a fluorescent or luminescent label, a radioactive label, an MRI (magnetic resonance imaging) or CT (electronic computer tomography) contrast agent, or an enzyme capable of producing a detectable product.

The therapeutic agents that can be bound or coupled with the antibody of the present invention include, but are not limited to: 1. a radionuclide; 2. a biological toxin; 3. a cytokine such as IL-2, etc.; 4. a gold nanoparticle/nanorod; 5. a viral particle; 6. a liposome; 7. a nanomagnetic particle; 8. a prodrug-activating enzyme (e. g., DT-diaphorase (DTD) or biphenyl hydrolase-like protein (BPHL)), etc.

### Pharmaceutical composition

The present invention also provides a composition. Preferably, the composition is a pharmaceutical composition comprising the above-mentioned fusion protein and/or the fusion protein complex molecule, and a pharmaceutically acceptable carrier. Typically, these substances may be formulated in a non-toxic, inert and pharmaceutically acceptable aqueous carrier medium, wherein the pH is typically about 5-8 and preferably about 6-8, although the pH may vary depending on the nature of the substance being formulated and the condition to be treated. The formulated pharmaceutical composition may be administered by conventional routes, including (but not limited to): intratumoral, intraperitoneal, intravenous, or topical administration.

The pharmaceutical composition of the present invention may be directly used to redirect CD8⁺T cells, and thus can be used to kill various tumor cells. In addition, other therapeutic agents may be used at the same time.

The pharmaceutical composition of the present invention contains a safe and effective amount (e.g., 0.001-99wt%, preferably 0.01-90wt%, more preferably 0.1-80wt%) of the above-mentioned fusion protein and/or the fusion protein complex molecule of the present invention (or the conjugate thereof) and a pharmaceutically acceptable carrier or excipient. Such carriers include (but are not limited to): saline, buffer, glucose, water, glycerol, ethanol, and combinations thereof. The pharmaceutical formulation should match the mode of administration. The pharmaceutical composition of the present invention may be prepared in the form of an injection, for example, by conventional methods using normal saline or aqueous solutions containing glucose and other adjuvants. The pharmaceutical composition such as an injection or solution should be manufactured under sterile conditions. The dosage of the active ingredient is a therapeutically effective amount, for example, about 10 µg/kg body weight per day to about 50 mg/kg body weight. In addition, the polypeptide of the present invention may also be used with other therapeutic agents.

When a pharmaceutical composition is used, a safe and effective amount of the fusion protein and/or the fusion protein complex molecule (or the conjugate thereof) is administered to a mammal, wherein the safe and effective amount is typically at least about 10 µg/kg body weight, and in most cases no more than about 50 mg/kg body weight, preferably about 10 µg/kg body weight to about 30 µg/kg body weight. Of course, the specific dosage should also consider factors such as the administration route and the patient's health status, which are all within the skill range of a skilled physician.

### Tumor

The term "tumor" refers to all types of cancer cell growth or carcinogenic processes, metastatic tissues or malignant transformed cells, tissues or organs, regardless of pathological type or stage of infection. Examples of tumors non-restrictively include: solid tumors, soft tissue tumors, and metastatic lesions. Examples of solid tumors include: malignant tumors of different organ systems, such as sarcoma, lung squamous cell carcinoma, and cancers. For example: infected prostate, lung, breast, lymph node, gastrointestinal tract (such as colon), and genitourinary tract (such as kidney, epithelial cells), and pharynx. Lung squamous cell carcinoma includes malignant tumors, such as most colon cancer, rectal cancer, renal cell carcinoma, liver cancer, non-small cell carcinoma of the lungs, small intestine cancer, and esophageal cancer. The metastatic lesions of the above-mentioned cancers can also be treated and prevented using the methods and compositions of the present invention.

### The main advantages of the present invention include:

(1) The fusion protein of the present invention avoids the introduction of flexible polypeptide linkers like scFV molecules, thus being closer to the natural state of natural antibody molecules.
(2) The fusion protein of the present invention reduces the risk of immunogenicity caused by the introduction of flexible polypeptide linkers.

The present invention is further explained below in conjunction with specific examples. It should be understood that these examples are only for illustrating the present invention and not intend to limit the scope of the present invention. The experimental method without detailed conditions specified in the following embodiments is generally in accordance with conventional conditions, such as those described in Sambrook J and Russell et al., "Molecular Cloning-A Laboratory Manual" (third edition, (2001) CSHL Press), or in accordance with the conditions recommended by the manufacturers. Unless otherwise stated, percentages and parts are calculated by weight.

The 1G4 TCR in Examples 1-7 can bind to the SLLMWITQC (SEQ ID NO: 37) short peptide presented on the HLA-A*0201 molecule.

### Example 1

### Preparation of a fusion protein (VH-TCR β form) formed by the fusion of the N-terminus of the 1G4 TCR β chain with the antibody heavy chain variable region

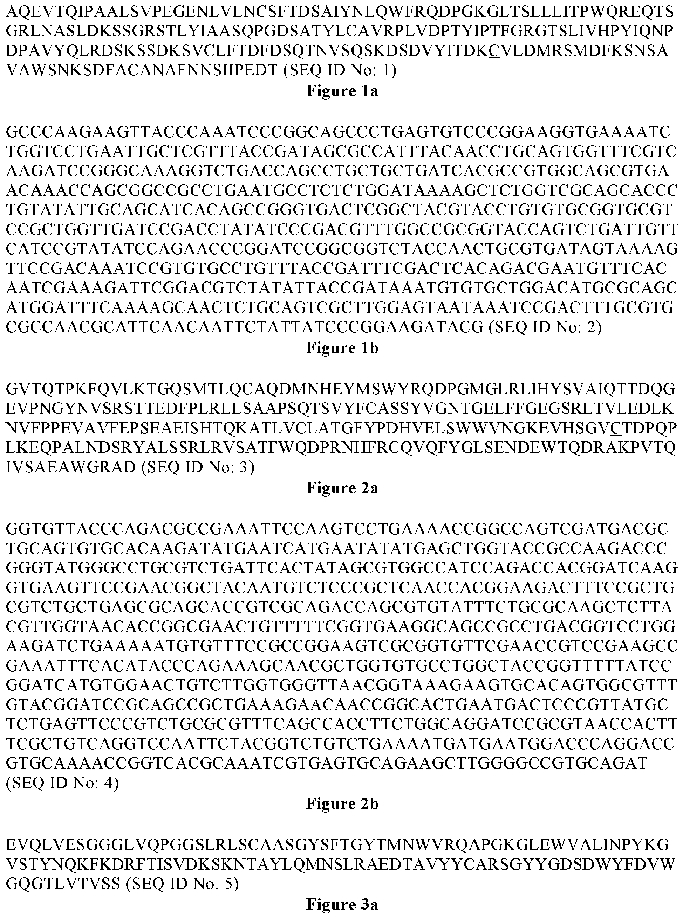

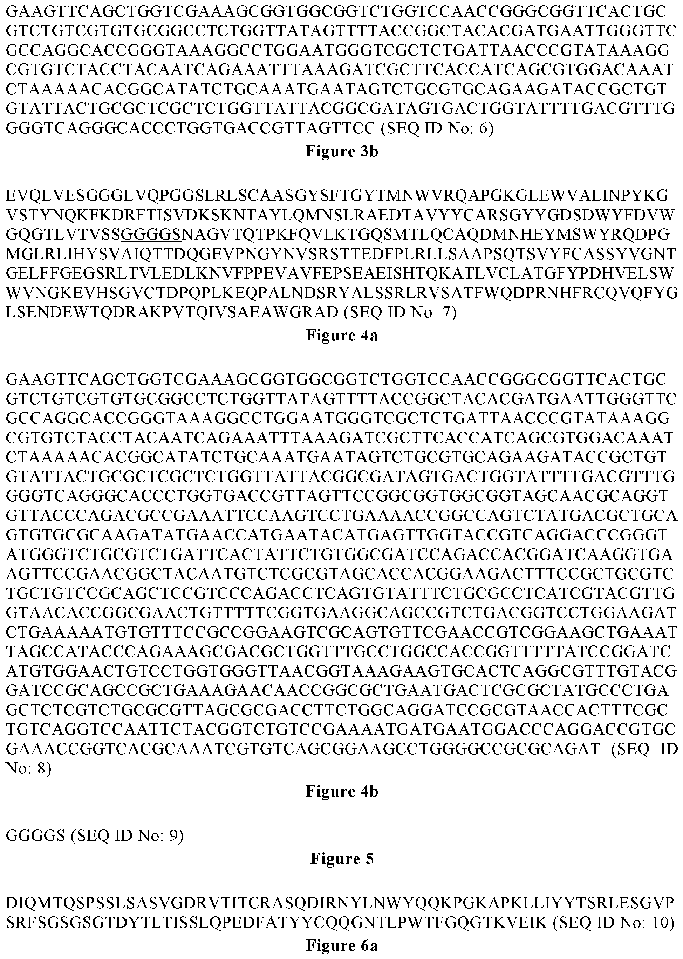

SEQ ID NO: 1 (Figure 1a) is the amino acid sequence of the 1G4 TCR α chain, wherein Thr47 in its TRAC constant region is substituted with Cys162.
SEQ ID NO: 2 (Figure 1b) is the nucleic acid sequence corresponding to SEQ ID NO: 1.
SEQ ID NO: 3 (Figure 2a) is the amino acid sequence of the 1G4 TCR β chain, wherein Ser56 in its TRBC constant region is substituted with Cys168.
SEQ ID NO: 4 (Figure 2b) is the nucleic acid sequence corresponding to SEQ ID NO: 3.
SEQ ID NO: 5 (Figure 3a) is the amino acid sequence of the antibody heavy chain variable region.
SEQ ID NO: 6 (Figure 3b) is the nucleic acid sequence corresponding to SEQ ID NO: 5.

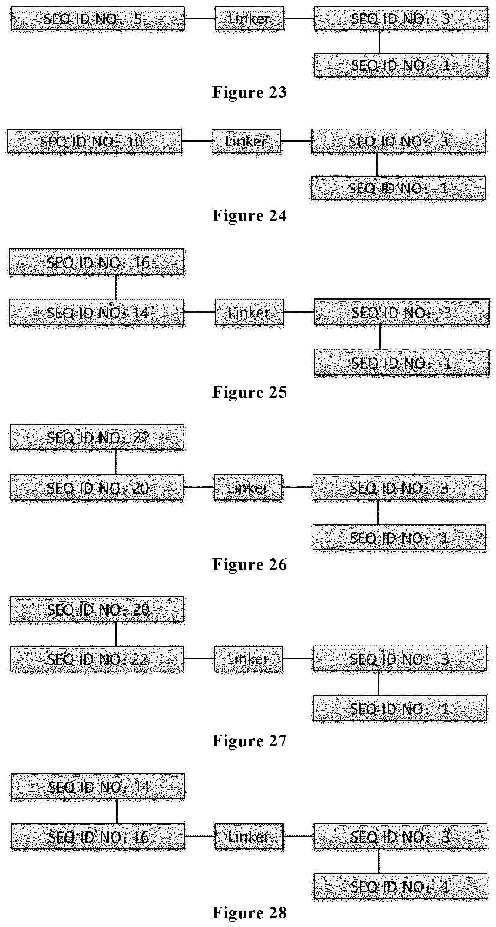

Figure 23 shows in block diagram form the structure of a multi-domain fusion molecule containing the α-chain shown in SEQ ID NO: 1 and the β-chain shown in SEQ ID NO: 3, and containing the antibody heavy chain variable region shown in SEQ ID NO: 5. The antibody heavy chain is fused to the N-terminus of the TCR β chain shown in SEQ ID NO: 3 via a linker with the sequence shown in SEQ ID NO: 7 (Figure 4a). The linker sequence is GGGGS (SEQ ID NO: 9).

SEQ ID NO: 8 (Figure 4b) is the nucleic acid sequence corresponding to SEQ ID NO: 7.

### Vector construction

The genes SEQ ID NO: 2 (Figure 1b) encoding the 1G4 TCR α-chain and SEQ ID NO: 8 (Figure 4b) encoding the antibody heavy chain fused to the 1G4 TCR β-chain were respectively cloned into the expression plasmid of pET-28a, which contained the T7 promoter to allow the high-level expression of the target genes in the *E. coli* BL21-DE3 strains.

### Expression

The constructed plasmids were individually transformed into *E. coli* strains BL21-DE3, and kanamycin-resistant monoclonal clones were grown in LB medium (50 µg/ml of kanamycin) at 37°C until the OD₆₀₀ was about 1.0, and then protein expression was induced with 1 mM IPTG. Three hours after induction, cells were collected by centrifugation at 4000 g for 15 min in a Thermo Scientific HERAEUS X1R centrifuge. Using 20 ml of BugBuster Master Mix (Merck Millipore), the organisms were resuspended by vortexing and shaking, and then after shaking and processing at room temperature for 20 min, they were placed in a pre-cooled high-speed centrifuge at 6000 g, 4°C, for 15 min, and then the supernatant was removed. 10 ml of BugBuster Master Mix was added again, the precipitate was resuspended by vortexing and shaking at room temperature for 5 min, then 30 ml of 10-fold diluted BugBuster (Merck Millipore) was added into it again, and the liquid was inverted up and down several times to mix well, then was put into the high-speed centrifuge again at 6000 g, 4 °C, for 15 min, and the supernatant was discarded. 30 ml of 10-fold diluted BugBuster was added, the precipitate was resuspended by vortexing and shaking, and the liquid was put into the high-speed centrifuge at 6000 g, 4°C for 15 min. The supernatant was discarded and the previous step was repeated twice. The supernatant was discarded, 30 ml of PBS was added to resuspend the inclusion bodies and centrifuged at 6000 g, 4°C for 15 min. After discarding the supernatant, 6 M of guanidine hydrochloride was added to dissolve the inclusion bodies. The purified inclusion bodies were serially diluted, then samples were subjected to SDS-PAGE for purity detection and yield estimation, and all the inclusion bodies were subjected to protein quantification, and were subdivided and transferred to -80°C for storage.

### Renaturation

The renaturation buffer for TCR (5 M urea, 100 mM Tris pH 8.1, 0.4 M L-arginine, 2 mM EDTA, 6.5 mM cysteamine, 1.87 mM cystamine) was prepared and pre-cooled to 4°C in advance. About 12 mg of TCR α chain and 11.2 mg of antibody heavy chain-TCR β chain were thawed from the cryopreservation medium. They were added to 6 ml of 6 M guanidine hydrochloride solution, respectively, and DTT at a final concentration of 15 mM was added. The mixture was mixed well, and then placed in a 37°C incubator for 40-min incubation. The incubated TCR α chain and heavy chain-TCR β chain inclusion bodies were added to the renaturation buffer of the TCR, respectively, and reacted for 30 min in a cold storage. A dialysis bag of 10 kDa was prepared and the reacted renaturation buffer was added into the dialysis bag, then the dialysis bag was put into pre-cooled deionized water and subjected to dialysis overnight in the cold storage. The next day, the dialysis bag was transferred to pre-cooled 10 mM Tris-HCl for dialysis. In the evening, the dialysis bag was again transferred to pre-cooled 10 mM Tris-HCl for dialysis overnight.

### Purification

Soluble and correctly folded multi-domain fusion molecules were separated from misfolding, degradation products and impurities by a 3-step purification method described below.

Anion exchange purification was used first. The dialyzed samples were placed in a pre-cooled high-speed centrifuge at 8000×g, 4°C for 15 min to remove the precipitate and the supernatant was again filtered through a 0.45 µm filter membrane. An anion-exchange column, HiTrap^{®} Q HP (GE Healthcare), was used to purify the renaturated samples. The column was rinsed with four column volumes of solution A (10 mM Tris-HCl, pH 8.0) and then the sample was loaded at a flow rate of 5 ml/min. After the sample was completely loaded, the column was again rinsed with solution A for about 4 column volumes, and when the conductivity and UV₂₈₀ both stabilized, a gradient elution was started, setting 0-100% of solution B (1M NaCl+10 mM Tris-HCl, pH 8.0) for 50 min, eluting the sample at a flow rate of 3 ml/min, and starting to collect when the UV₂₈₀ was significantly elevated, 1 ml was collected from each tube. The peak components were analyzed by 12% SDS-PAGE and then combined.

This was followed by purification using molecular sieves (Superdex 75, GE Healthcare). The sample purified in the previous step was concentrated using a 10 kDa ultrafiltration tube at 3500×g, 4°C to 500 µl, followed by molecular sieve purification. The molecular sieves were equilibrated first with deionized water and then equilibrated again with PBS. The sample loop was washed with 500 µl of PBS and the sample was loaded, and after the sample was loaded, the sample was again equilibrated and eluted with PBS at a flow rate of 1 ml/min, and 0.4 ml was collected from each tube. The peak components were analyzed by 12% SDS-PAGE and then combined.

A final anion-exchange purification was performed using an anion-exchange column, HiTrap^{®} Q HP (GE Healthcare). The samples purified in the previous step were diluted 20-fold using pre-cooled 10 mM Tris and then loaded. The column was rinsed first with four column volumes of solution A, and then the sample was loaded at a flow rate of 5 ml/min. After the sample was fully loaded, the column was again rinsed with solution A for about 4 column volumes. When both conductivity and UV₂₈₀ stabilized, a gradient elution was started, setting 0-100% of solution B for 50 min, eluting the sample at a flow rate of 3 ml/min, and collecting the sample when the UV₂₈₀ was significantly elevated, with 1 ml collected from each tube. The peak components were analyzed by 12% SDS-PAGE and then combined. The samples were concentrated using a 10 kDa ultrafiltration tube at 3500×g, 4 °C to 500 µl and the buffer was replaced using PBS buffer and the concentration was determined, then subdivided and stored at -80 °C.

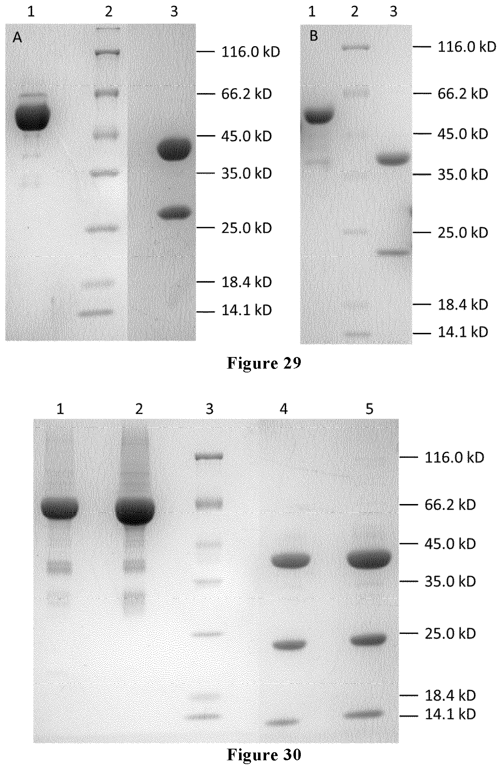

The SDS-PAGE profile of the purified protein was shown in Figure 29 (A): Lane 1: fusion protein (VH-TCR β form) in non-reduced state formed by the fusion of the N-terminus of the 1G4 TCR β chain with the antibody heavy chain variable region; Lane 2: marker; Lane 3: fusion protein (VH-TCR β form) in reduced state formed by the fusion of the N-terminus of the 1G4 TCR β chain with the antibody heavy chain variable region.

### Example 2

### Preparation of a fusion molecule (VL-TCR β form) formed by the fusion of the N-terminus of the 1G4 TCR β chain with the antibody light chain variable region

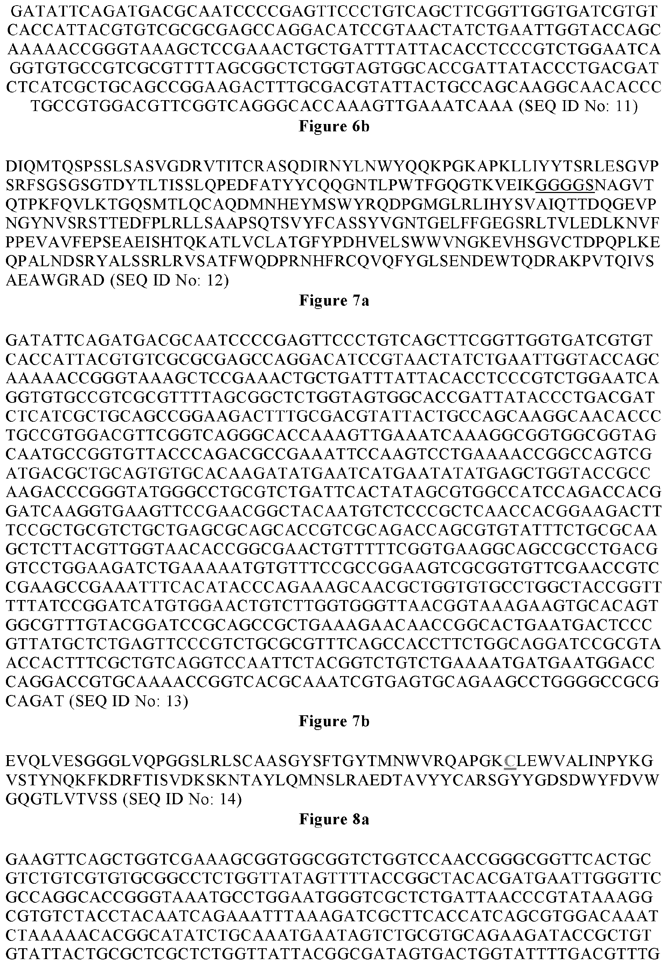

SEQ ID NO: 1 (Figure 1a) is the amino acid sequence of the 1G4 TCR α chain, wherein Thr47 in its TRAC constant region is substituted with Cys162.
SEQ ID NO: 2 (Figure 1b) is the nucleic acid sequence corresponding to SEQ ID NO: 1.
SEQ ID NO: 3 (Figure 2a) is the amino acid sequence of the 1G4 TCR β chain, wherein Ser56 in its TRBC constant region is substituted with Cys168.
SEQ ID NO: 4 (Figure 2b) is the nucleic acid sequence corresponding to SEQ ID NO: 3.
SEQ ID NO: 10 (Figure 6a) is the amino acid sequence of the antibody light chain variable region.
SEQ ID NO: 11 (Figure 6b) is the nucleic acid sequence corresponding to SEQ ID NO: 10.

Figure 24 shows in block diagram form the structure of a multi-domain fusion molecule containing the α-chain shown in SEQ ID NO: 1 and the β-chain shown in SEQ ID NO: 3, and containing the antibody light chain variable region shown in SEQ ID NO: 10. The antibody light chain is fused to the N-terminus of the TCR β chain shown in SEQ ID NO: 3 via a linker with the sequence shown in SEQ ID NO: 12 (Figure 7a). The linker sequence is GGGGS (SEQ ID NO: 9).

SEQ ID NO: 13 (Figure 7b) is the nucleic acid sequence corresponding to SEQ ID NO: 12.

### Vector construction

The genes SEQ ID NO: 2 (Figure 1b) encoding the 1G4 TCR α-chain and SEQ ID NO: 13 (Figure 7b) encoding the antibody light chain fused to the 1G4 TCR β-chain were respectively cloned into the expression plasmid of pET-28a, which contained the T7 promoter to allow the high-level expression of the target genes in the *E. coli* BL21-DE3 strains.

### Expression

The constructed plasmids were individually transformed into *E. coli* strains BL21-DE3, and kanamycin-resistant monoclonal clones were grown in LB medium (50 µg/ml of kanamycin) at 37°C until the OD₆₀₀ was about 1.0, and then protein expression was induced with 1 mM IPTG. Three hours after induction, cells were collected by centrifugation at 4000 g for 15 min in a Thermo Scientific HERAEUS X1R centrifuge. Using 20 ml of BugBuster Master Mix (Merck Millipore), the organisms were resuspended by vortexing and shaking, and then after shaking and processing at room temperature for 20 min, they were placed in a pre-cooled high-speed centrifuge at 6000 g, 4°C, for 15 min, and then the supernatant was removed. 10 ml of BugBuster Master Mix was added again, the precipitate was resuspended by vortexing and shaking at room temperature for 5 min, then 30 ml of 10-fold diluted BugBuster (Merck Millipore) was added into it again, and the liquid was inverted up and down several times to mix well, then was put into the high-speed centrifuge again at 6000 g, 4 °C, for 15 min, and the supernatant was discarded. 30 ml of 10-fold diluted BugBuster was added, the precipitate was resuspended by vortexing and shaking, and the liquid was put into the high-speed centrifuge at 6000 g, 4°C for 15 min. The supernatant was discarded and the previous step was repeated twice. The supernatant was discarded, 30 ml of PBS was added to resuspend the inclusion bodies and centrifuged at 6000 g, 4°C for 15 min. After discarding the supernatant, 6 M of guanidine hydrochloride was added to dissolve the inclusion bodies. The purified inclusion bodies were serially diluted, then samples were subjected to SDS-PAGE for purity detection and yield estimation, and all the inclusion bodies were subjected to protein quantification, and were subdivided and transferred to -80°C for storage.

### Renaturation

The renaturation buffer for TCR (5 M urea, 100 mM Tris pH 8.1, 0.4 M L-arginine, 2 mM EDTA, 6.5 mM cysteamine, 1.87 mM cystamine) was prepared and pre-cooled to 4°C in advance. About 12 mg of TCR α chain and 11.2 mg of antibody light chain-TCR β chain were thawed from the cryopreservation medium. They were added to 6 ml of 6 M guanidine hydrochloride solution, respectively, and DTT at a final concentration of 15 mM was added. The mixture was mixed well, and then placed in a 37°C incubator for 40-min incubation. The incubated TCR α chain and light chain-TCR β chain inclusion bodies were added to the renaturation buffer of the TCR, respectively, and reacted for 30 min in a cold storage. A dialysis bag of 10 kDa was prepared and the reacted renaturation buffer was added into the dialysis bag, then the dialysis bag was put into pre-cooled deionized water and subjected to dialysis overnight in the cold storage. The next day, the dialysis bag was transferred to pre-cooled 10 mM Tris-HCl for dialysis. In the evening, the dialysis bag was again transferred to pre-cooled 10 mM Tris-HCl for dialysis overnight.

### Purification

Soluble and correctly folded multi-domain fusion molecules were separated from misfolding, degradation products and impurities by a 3-step purification method described below.

Anion exchange purification was used first. The dialyzed samples were placed in a pre-cooled high-speed centrifuge at 8000×g, 4°C for 15 min to remove the precipitate and the supernatant was again filtered through a 0.45 µm filter membrane. An anion-exchange column, HiTrap^{®} Q HP (GE Healthcare), was used to purify the renaturated samples. The column was rinsed with four column volumes of solution A (10 mM Tris-HCl, pH 8.0) and then the sample was loaded at a flow rate of 5 ml/min. After the sample was completely loaded, the column was again rinsed with solution A for about 4 column volumes, and when the conductivity and UV₂₈₀ both stabilized, a gradient elution was started, setting 0-100% of solution B (1M NaCl+10 mM Tris-HCl, pH 8.0) for 50 min, eluting the sample at a flow rate of 3 ml/min, and starting to collect when the UV₂₈₀ was significantly elevated, 1 ml was collected from each tube. The peak components were analyzed by 12% SDS-PAGE and then combined.

This was followed by purification using molecular sieves (Superdex 75, GE Healthcare). The sample purified in the previous step was concentrated using a 10 kDa ultrafiltration tube at 3500×g, 4°C to 500 µl, followed by molecular sieve purification. The molecular sieves were equilibrated first with deionized water and then equilibrated again with PBS. The sample loop was washed with 500 µl of PBS and the sample was loaded, and after the sample was loaded, the sample was again equilibrated and eluted with PBS at a flow rate of 1 ml/min, and 0.4 ml was collected from each tube. The peak components were analyzed by 12% SDS-PAGE and then combined.

A final anion-exchange purification was performed using an anion-exchange column, HiTrap^{®} Q HP (GE Healthcare). The samples purified in the previous step were diluted 20-fold using pre-cooled 10 mM Tris and then loaded. The column was rinsed first with four column volumes of solution A, and then the sample was loaded at a flow rate of 5 ml/min. After the sample was fully loaded, the column was again rinsed with solution A for about 4 column volumes. When both conductivity and UV₂₈₀ stabilized, a gradient elution was started, setting 0-100% of solution B for 50 min, eluting the sample at a flow rate of 3 ml/min, and collecting the sample when the UV₂₈₀ was significantly elevated, with 1 ml collected from each tube. The peak components were analyzed by 12% SDS-PAGE and then combined. The samples were concentrated using a 10 kDa ultrafiltration tube at 3500×g, 4 °C to 500 µl and the buffer was replaced using PBS buffer and the concentration was determined, then subdivided and stored at -80 °C.

The SDS-PAGE profile of the purified protein was shown in Figure 29 (B): Lane 1: fusion protein (VL-TCR β form) in non-reduced state formed by the fusion of the N-terminus of the 1G4 TCR β chain with the antibody light chain variable region; Lane 2: marker; Lane 3: fusion protein (VL-TCR β form) in reduced state formed by the fusion of the N-terminus of the 1G4 TCR β chain with the antibody light chain variable region.

### Example 3

### Properties of a fusion molecule (VH-TCR β form) formed by the fusion of the N-terminus of the 1G4 TCR β chain with the antibody heavy chain variable region and a fusion molecule (VL-TCR β form) formed by the fusion of the N-terminus of the 1G4 TCR β chain with the antibody light chain variable region

The ability of bifunctional fusion molecules to redirect T cells to specifically kill tumor cells has been reported in several studies. The basic principle is that they can mimic the key signals of T cell activation to exert effector functions. On the one hand, they can recognize the pMHC complex on the surface of tumor cells through their specific TCRs with high affinity; and on the other hand, they can activate the downstream signaling pathways of T cell activation through the CD3 antibody end, so as to direct the T cells to specifically kill the tumor cells.

The following embodiments demonstrate that the fusion molecules of the present invention which only fused the antibody heavy chain variable region or antibody light chain variable region are not able to direct CD8⁺ T cells to kill target cell lines. This assay is a colorimetric assay alternative to the 51Cr release cytotoxicity assay that quantifies lactate dehydrogenase (LDH) released upon cell lysis. A 30-minute coupled enzyme reaction was used to detect LDH released in the medium, and in the enzyme reaction LDH converts a tetrazolium salt (INT) to a red colored formanzan. The amount of red product generated is proportional to the number of cells lysed. Data for visible light absorption values at 490 nm can be collected using a standard 96-well plate reader.

Those skilled in the art are familiar with methods for detecting cell function using LDH release assays. The target cell lines selected for LDH experiments in this embodiment are MDA-MB-231 (NY-ESO-1 positive, A2 positive, breast cancer cell line), MDA-MB-231 (NY-ESO-1 negative, A2 positive, breast cancer cell line), and NCI-H1299-A2 (NY-ESO-1 positive, A2 positive, lung cancer cell line), NCI-H1299 (NY-ESO-1 positive, A2 negative, lung cancer cell line).

CD8⁺ T cells were used as effector cells, and MDA-MB-231 (NY-ESO-1 positive, A2 positive, breast cancer cell line) and NCI-H1299 (NY-ESO-1 positive, A2 positive, lung cancer cell line) were used as target cells, and the E : T ratio selected was 5:1. Both the desired effector and target cells were diluted well in culture medium according to the desired number of cells, i.e. the cell density of the desired effector cells was 1×10⁶ /ml, while the cell density of the desired target cells was 2×10⁵ /ml. MDA-MB-231 (NY-ESO-1 negative, A2 positive, breast cancer cell line) and NCI-H1299 (NY-ESO-1 positive, A2 negative, lung cancer cell line) were also used as negative control target cells.

When the NCI-H1299 lung cancer cell line or the MDA-MB-231 breast cancer cell line was used as a target cell, the proteins formed by the fusion of the N-terminus of the 1G4 TCR β chain with the antibody heavy chain variable region and a fusion molecule (VL-TCR β form) formed by the fusion of the N-terminus of the 1G4 TCR β chain with the antibody light chain variable region were diluted with the culture medium to 4×10⁻⁷ M, 4×10⁻⁸ M, 4×10⁻⁹ M, 4×10⁻¹⁰ M, 4×10⁻¹¹ M, and 4×10⁻¹² M.

A 96-well round-bottom plate was prepared and 50 µl of effector cells, 50 µl of multi-domain bifunctional fusion molecule proteins and 100 µl of target cells were added to the wells, and the target cell maximal lysis wells, the target cell spontaneous wells, the effector cell spontaneous wells, the medium spontaneous wells, and the medium plus the lysate spontaneous wells were set up with a final volume of 200 µl for each well, and each of them has three duplicate wells. The 96-well round-bottom plates were incubated in a cell culture incubator at 37°C, 5% CO₂ for 24 h. After that, the 96-well round-bottom plates were put into a centrifuge at 250×g for 4 min. 50 µl of supernatant was taken out to the 96-well flat-bottomed plates and 50 µl of the substrate solution was added, and the reaction was carried out at ambient temperature under the protection from light for 30 min. After the reaction was finished, 50 µl of termination solution was added and the absorption value was measured at 490 nm by a microplate reader immediately. The specific killing efficiency was calculated according to the instruction manual = (experimental wells - effector cell spontaneous wells - target cell spontaneous wells) / (target cell maximum lysis wells - target cell spontaneous wells).

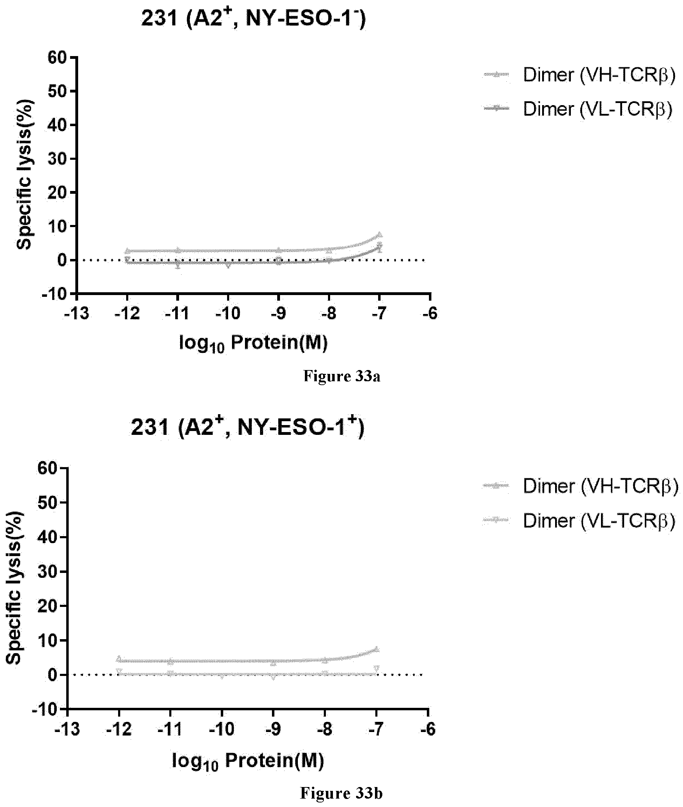

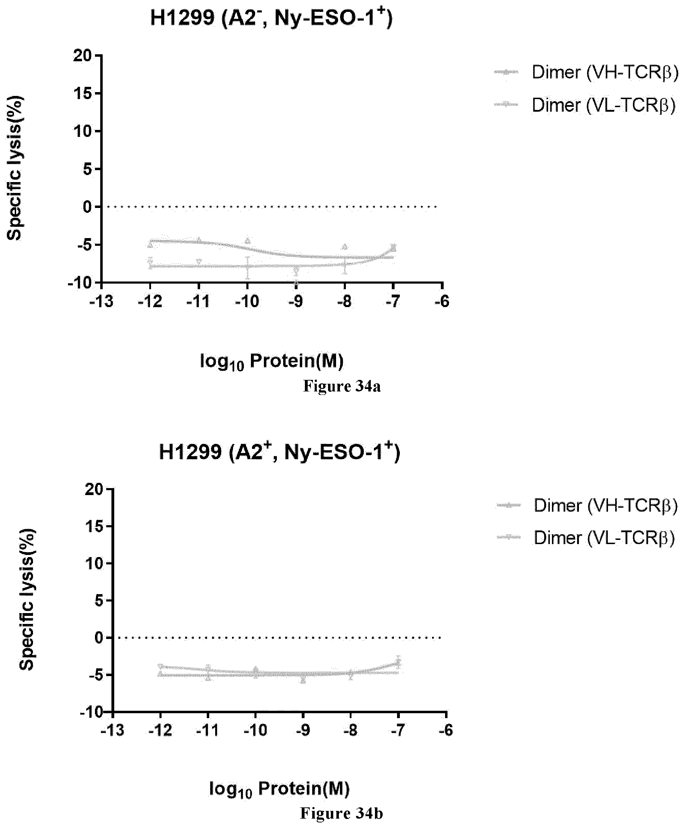

The results, as shown in Figure 33a, Figure 33b and Figure 34a, Figure 34b, showed that the fusion molecules which only fused the antibody heavy chain variable region or antibody light chain variable region were not able to direct CD8⁺ T cells to kill the MDA-MB-231 (NY-ESO-1 positive, A2 positive, breast cancer cell line) and NCI-H1299 (NY-ESO-1 positive, A2 positive, lung cancer cell line), nor were they able to kill the MDA-MB-231 (NY-ESO-1 negative, A2 positive, breast cancer cell line) and the NCI-H1299 (NY-ESO-1 positive, A2 negative, lung cancer cell line).

### Example 4

### Preparation of a multi-domain fusion protein complex molecule (VH44-TCR β form) formed by the fusion of the N-terminus of the 1G4 TCR β chain with the antibody heavy chain variable region

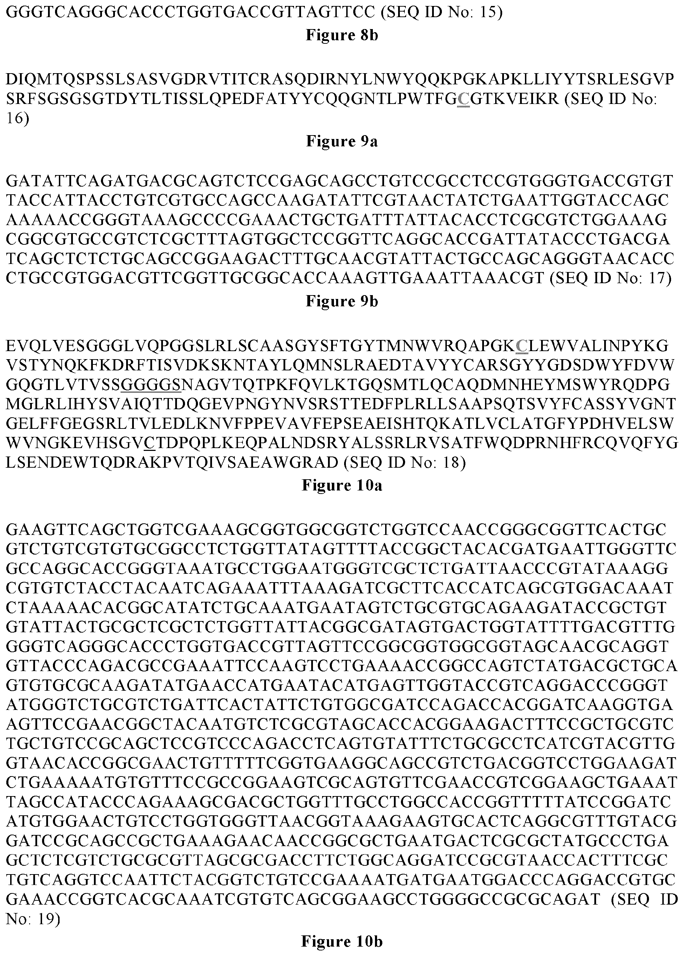

SEQ ID NO: 1 (Figure 1a) is the amino acid sequence of the 1G4 TCR α chain, wherein Thr47 in its TRAC constant region is substituted with Cys162.
SEQ ID NO: 2 (Figure 1b) is the nucleic acid sequence corresponding to SEQ ID NO: 1.
SEQ ID NO: 3 (Figure 2a) is the amino acid sequence of the 1G4 TCR β chain, wherein Ser56 in its TRBC constant region is substituted with Cys168.
SEQ ID NO: 4 (Figure 2b) is the nucleic acid sequence corresponding to SEQ ID NO: 3.
SEQ ID NO: 14 (Figure 8a) is the sequence obtained by replacing Gly44 in the original sequence with Cys44 in the amino acid sequence of the antibody heavy chain variable region.
SEQ ID NO: 15 (Figure 8b) is the nucleic acid sequence corresponding to SEQ ID NO: 14.
SEQ ID NO: 16 (Figure 9a) is the sequence obtained by replacing Gln100 in the original sequence with Cys100 in the amino acid sequence of the antibody light chain variable region.
SEQ ID NO: 17 (Figure 9b) is the nucleic acid sequence corresponding to SEQ ID NO: 16.

Figure 25 shows in block diagram form the structure of a multi-domain fusion molecule containing the α-chain shown in SEQ ID NO: 1 and the β-chain shown in SEQ ID NO: 3, and containing the antibody heavy chain variable region shown in SEQ ID NO: 14 and the antibody light chain variable region shown in SEQ ID NO: 16. The antibody heavy chain is fused to the N-terminus of the TCR β chain shown in SEQ ID NO: 3 via a linker with the sequence shown in SEQ ID NO: 18 (Figure 10a). The linker sequence is GGGGS (SEQ ID NO: 9).

SEQ ID NO: 19 (Figure 10b) is the nucleic acid sequence corresponding to SEQ ID NO: 18.

### Vector construction

The genes SEQ ID NO: 2 (Figure 1b) encoding the 1G4 TCR α-chain and SEQ ID NO: 19 (Figure 10b) encoding the antibody heavy chain fused to the 1G4 TCR β-chain, and SEQ ID NO: 17 (Figure 9b) encoding the antibody light chain were respectively cloned into the expression plasmid of pET-28a, which contained the T7 promoter to allow the high-level expression of the target genes in the *E. coli* BL21-DE3 strains.

### Expression

The constructed plasmids were individually transformed into *E. coli* strains BL21-DE3, and kanamycin-resistant monoclonal clones were grown in LB medium (50 µg/ml of kanamycin) at 37°C until the OD₆₀₀ was about 1.0, and then protein expression was induced with 1 mM IPTG. Three hours after induction, cells were collected by centrifugation at 4000 g for 15 min in a Thermo Scientific HERAEUS X1R centrifuge. Using 20 ml of BugBuster Master Mix (Merck Millipore), the organisms were resuspended by vortexing and shaking, and then after shaking and processing at room temperature for 20 min, they were placed in a pre-cooled high-speed centrifuge at 6000 g, 4°C, for 15 min, and then the supernatant was removed. 10 ml of BugBuster Master Mix was added again, the precipitate was resuspended by vortexing and shaking at room temperature for 5 min, then 30 ml of 10-fold diluted BugBuster (Merck Millipore) was added into it again, and the liquid was inverted up and down several times to mix well, then was put into the high-speed centrifuge again at 6000 g, 4 °C, for 15 min, and the supernatant was discarded. 30 ml of 10-fold diluted BugBuster was added, the precipitate was resuspended by vortexing and shaking, and the liquid was put into the high-speed centrifuge at 6000 g, 4°C for 15 min. The supernatant was discarded and the previous step was repeated twice. The supernatant was discarded, 30 ml of PBS was added to resuspend the inclusion bodies and centrifuged at 6000 g, 4°C for 15 min. After discarding the supernatant, 6 M of guanidine hydrochloride was added to dissolve the inclusion bodies. The purified inclusion bodies were serially diluted, then samples were subjected to SDS-PAGE for purity detection and yield estimation, and all the inclusion bodies were subjected to protein quantification, and were subdivided and transferred to -80°C for storage.

### Renaturation

The renaturation buffer for TCR (5 M urea, 100 mM Tris pH 8.1, 0.4 M L-arginine, 2 mM EDTA, 6.5 mM cysteamine, 1.87 mM cystamine) was prepared and pre-cooled to 4°C in advance. About 12 mg of TCR α chain and 11.2 mg of antibody heavy chain-TCR β chain, and 6 mg of antibody light chain inclusion bodies were thawed from the cryopreservation medium. They were added to 6 ml of 6 M guanidine hydrochloride solution, respectively, and DTT at a final concentration of 15 mM was added. The mixture was mixed well, and then placed in a 37°C incubator for 40-min incubation. The incubated TCR α chain and heavy chain-TCR β chain and antibody light chain inclusion bodies were added to the renaturation buffer of the TCR, respectively, and reacted for 30 min in a cold storage. A dialysis bag of 10 kDa was prepared and the reacted renaturation buffer was added into the dialysis bag, then the dialysis bag was put into pre-cooled deionized water and subjected to dialysis overnight in the cold storage. The next day, the dialysis bag was transferred to pre-cooled 10 mM Tris-HCl for dialysis. In the evening, the dialysis bag was again transferred to pre-cooled 10 mM Tris-HCl for dialysis overnight.

### Purification

Soluble and correctly folded multi-domain fusion molecules were separated from misfolding, degradation products and impurities by a 3-step purification method described below.

Anion exchange purification was used first. The dialyzed samples were placed in a pre-cooled high-speed centrifuge at 8000×g, 4°C for 15 min to remove the precipitate and the supernatant was again filtered through a 0.45 µm filter membrane. An anion-exchange column, HiTrap^{®} Q HP (GE Healthcare), was used to purify the renaturated samples. The column was rinsed with four column volumes of solution A (10 mM Tris-HCl, pH 8.0) and then the sample was loaded at a flow rate of 5 ml/min. After the sample was completely loaded, the column was again rinsed with solution A for about 4 column volumes, and when the conductivity and UV₂₈₀ both stabilized, a gradient elution was started, setting 0-100% of solution B (1M NaCl+10 mM Tris-HCl, pH 8.0) for 50 min, eluting the sample at a flow rate of 3 ml/min, and starting to collect when the UV₂₈₀ was significantly elevated, 1 ml was collected from each tube. The peak components were analyzed by 12% SDS-PAGE and then combined.

This was followed by purification using molecular sieves (Superdex 75, GE Healthcare). The sample purified in the previous step was concentrated using a 10 kDa ultrafiltration tube at 3500×g, 4°C to 500 µl, followed by molecular sieve purification. The molecular sieves were equilibrated first with deionized water and then equilibrated again with PBS. The sample loop was washed with 500 µl of PBS and the sample was loaded, and after the sample was loaded, the sample was again equilibrated and eluted with PBS at a flow rate of 1 ml/min, and 0.4 ml was collected from each tube. The peak components were analyzed by 12% SDS-PAGE and then combined.

A final anion-exchange purification was performed using an anion-exchange column, HiTrap^{®} Q HP (GE Healthcare). The samples purified in the previous step were diluted 20-fold using pre-cooled 10 mM Tris and then loaded. The column was rinsed first with four column volumes of solution A, and then the sample was loaded at a flow rate of 5 ml/min. After the sample was fully loaded, the column was again rinsed with solution A for about 4 column volumes. When both conductivity and UV₂₈₀ stabilized, a gradient elution was started, setting 0-100% of solution B for 50 min, eluting the sample at a flow rate of 3 ml/min, and collecting the sample when the UV₂₈₀ was significantly elevated, with 1 ml collected from each tube. The peak components were analyzed by 12% SDS-PAGE and then combined. The samples were concentrated using a 10 kDa ultrafiltration tube at 3500×g, 4 °C to 500 µl and the buffer was replaced using PBS buffer and the concentration was determined, then subdivided and stored at -80 °C.

The SDS-PAGE profile of the purified protein was shown in Figure 30: Lane 1: multi-domain bifunctional fusion molecule (VH44-TCR β form) in non-reduced state; Lane 3: marker; Lane 4: multi-domain bifunctional fusion molecule (VH44-TCR β form) in reduced state.

### Example 5

### Preparation of a multi-domain fusion protein complex molecule (VH105-TCR β form) formed by the fusion of the N-terminus of the 1G4 TCR β chain with the antibody heavy chain variable region

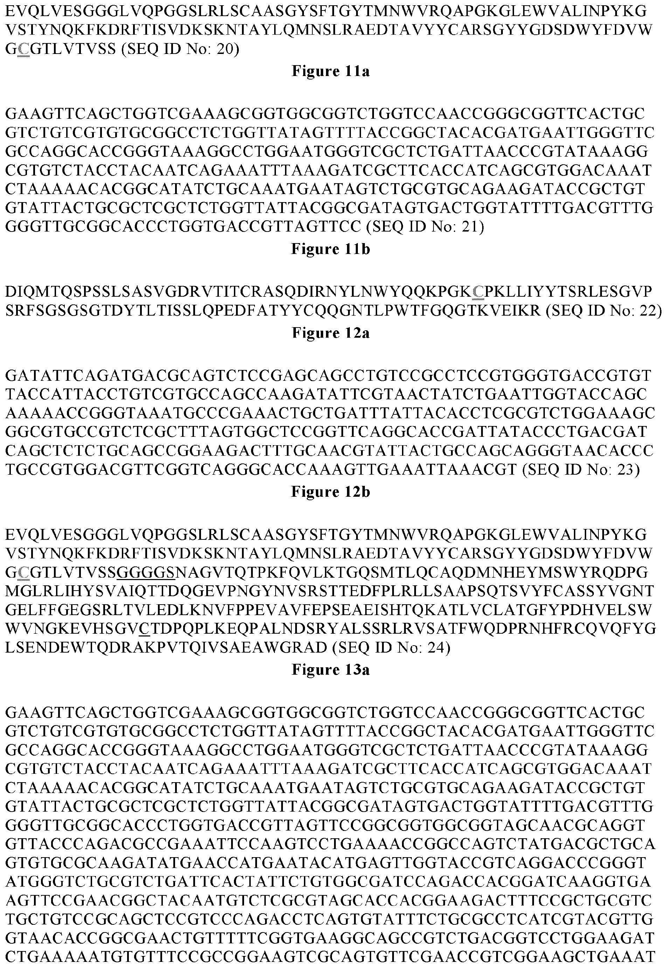

SEQ ID NO: 1 (Figure 1a) is the amino acid sequence of the 1G4 TCR α chain, wherein Thr47 in its TRAC constant region is substituted with Cys162.
SEQ ID NO: 2 (Figure 1b) is the nucleic acid sequence corresponding to SEQ ID NO: 1.
SEQ ID NO: 3 (Figure 2a) is the amino acid sequence of the 1G4 TCR β chain, wherein Ser56 in its TRBC constant region is substituted with Cys168.
SEQ ID NO: 4 (Figure 2b) is the nucleic acid sequence corresponding to SEQ ID NO: 3.
SEQ ID NO: 20 (Figure 11a) is the sequence obtained by replacing Gln105 in the original sequence with Cys105 in the amino acid sequence of the antibody heavy chain variable region.
SEQ ID NO: 21 (Figure 11b) is the nucleic acid sequence corresponding to SEQ ID NO: 20.
SEQ ID NO: 22 (Figure 12a) is the sequence obtained by replacing Ala43 in the original sequence with Cys43 in the amino acid sequence of the antibody light chain variable region.
SEQ ID NO: 23 (Figure 12b) is the nucleic acid sequence corresponding to SEQ ID NO: 22.

Figure 26 shows in block diagram form the structure of a multi-domain fusion molecule containing the α-chain shown in SEQ ID NO: 1 and the β-chain shown in SEQ ID NO: 3, and containing the antibody heavy chain shown in SEQ ID NO: 20 and the antibody light chain shown in SEQ ID NO: 22. The antibody heavy chain is fused to the N-terminus of the TCR β chain shown in SEQ ID NO: 3 via a linker with the sequence shown in SEQ ID NO: 24 (Figure 13a). The linker sequence is GGGGS (SEQ ID NO: 9).

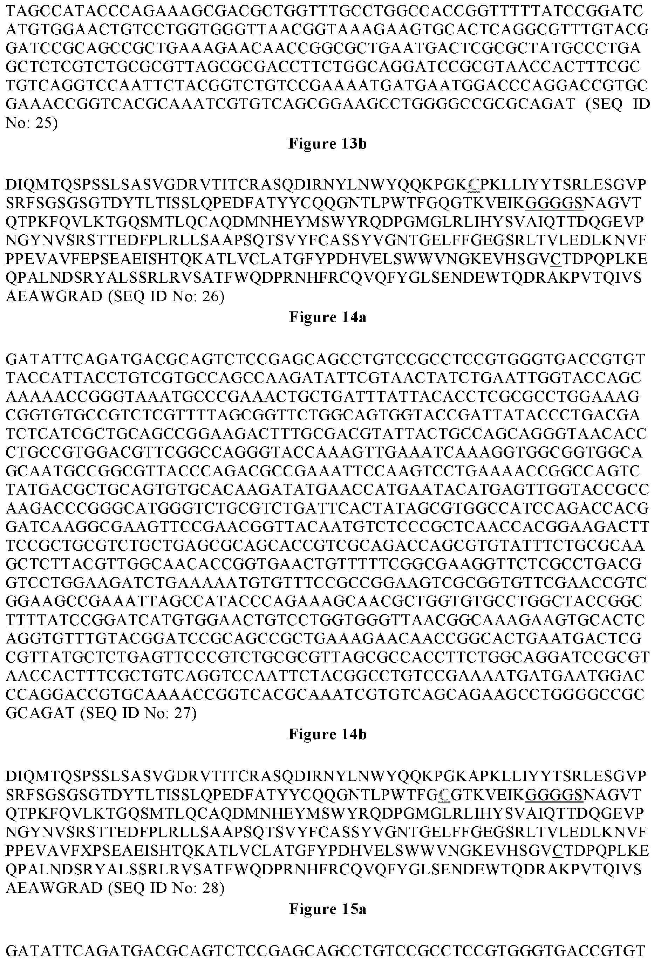

SEQ ID NO: 25 (Figure 13b) is the nucleic acid sequence corresponding to SEQ ID NO: 24.

### Vector construction

The genes SEQ ID NO: 2 (Figure 1b) encoding the 1G4 TCR α-chain and SEQ ID NO: 25 (Figure 13b) encoding the antibody heavy chain fused to the 1G4 TCR β-chain, and SEQ ID NO: 23 (Figure 12b) encoding the antibody light chain were respectively cloned into the expression plasmid of pET-28a, which contained the T7 promoter to allow the high-level expression of the target genes in the *E. coli* BL21-DE3 strains.

### Expression

The constructed plasmids were individually transformed into *E. coli* strains BL21-DE3, and kanamycin-resistant monoclonal clones were grown in LB medium (50 µg/ml of kanamycin) at 37°C until the OD₆₀₀ was about 1.0, and then protein expression was induced with 1 mM IPTG. Three hours after induction, cells were collected by centrifugation at 4000 g for 15 min in a Thermo Scientific HERAEUS X1R centrifuge. Using 20 ml of BugBuster Master Mix (Merck Millipore), the organisms were resuspended by vortexing and shaking, and then after shaking and processing at room temperature for 20 min, they were placed in a pre-cooled high-speed centrifuge at 6000 g, 4°C, for 15 min, and then the supernatant was removed. 10 ml of BugBuster Master Mix was added again, the precipitate was resuspended by vortexing and shaking at room temperature for 5 min, then 30 ml of 10-fold diluted BugBuster (Merck Millipore) was added into it again, and the liquid was inverted up and down several times to mix well, then was put into the high-speed centrifuge again at 6000 g, 4 °C, for 15 min, and the supernatant was discarded. 30 ml of 10-fold diluted BugBuster was added, the precipitate was resuspended by vortexing and shaking, and the liquid was put into the high-speed centrifuge at 6000 g, 4°C for 15 min. The supernatant was discarded and the previous step was repeated twice. The supernatant was discarded, 30 ml of PBS was added to resuspend the inclusion bodies and centrifuged at 6000 g, 4°C for 15 min. After discarding the supernatant, 6 M of guanidine hydrochloride was added to dissolve the inclusion bodies. The purified inclusion bodies were serially diluted, then samples were subjected to SDS-PAGE for purity detection and yield estimation, and all the inclusion bodies were subjected to protein quantification, and were subdivided and transferred to -80°C for storage.

### Renaturation

The renaturation buffer for TCR (5 M urea, 100 mM Tris pH 8.1, 0.4 M L-arginine, 2 mM EDTA, 6.5 mM cysteamine, 1.87 mM cystamine) was prepared and pre-cooled to 4°C in advance. About 12 mg of TCR α chain and 11.2 mg of antibody heavy chain-TCR β chain, and 6 mg of antibody light chain inclusion bodies were thawed from the cryopreservation medium. They were added to 6 ml of 6 M guanidine hydrochloride solution, respectively, and DTT at a final concentration of 15 mM was added. The mixture was mixed well, and then placed in a 37°C incubator for 40-min incubation. The incubated TCR α chain and heavy chain-TCR β chain and antibody light chain inclusion bodies were added to the renaturation buffer of the TCR, respectively, and reacted for 30 min in a cold storage. A dialysis bag of 10 kDa was prepared and the reacted renaturation buffer was added into the dialysis bag, then the dialysis bag was put into pre-cooled deionized water and subjected to dialysis overnight in the cold storage. The next day, the dialysis bag was transferred to pre-cooled 10 mM Tris-HCl for dialysis. In the evening, the dialysis bag was again transferred to pre-cooled 10 mM Tris-HCl for dialysis overnight.

### Purification

Soluble and correctly folded multi-domain fusion molecules were separated from misfolding, degradation products and impurities by a 3-step purification method described below.

Anion exchange purification was used first. The dialyzed samples were placed in a pre-cooled high-speed centrifuge at 8000×g, 4°C for 15 min to remove the precipitate and the supernatant was again filtered through a 0.45 µm filter membrane. An anion-exchange column, HiTrap^{®} Q HP (GE Healthcare), was used to purify the renaturated samples. The column was rinsed with four column volumes of solution A (10 mM Tris-HCl, pH 8.0) and then the sample was loaded at a flow rate of 5 ml/min. After the sample was completely loaded, the column was again rinsed with solution A for about 4 column volumes, and when the conductivity and UV₂₈₀ both stabilized, a gradient elution was started, setting 0-100% of solution B (1M NaCl+10 mM Tris-HCl, pH 8.0) for 50 min, eluting the sample at a flow rate of 3 ml/min, and starting to collect when the UV₂₈₀ was significantly elevated, 1 ml was collected from each tube. The peak components were analyzed by 12% SDS-PAGE and then combined.

This was followed by purification using molecular sieves (Superdex 75, GE Healthcare). The sample purified in the previous step was concentrated using a 10 kDa ultrafiltration tube at 3500×g, 4°C to 500 µl, followed by molecular sieve purification. The molecular sieves were equilibrated first with deionized water and then equilibrated again with PBS. The sample loop was washed with 500 µl of PBS and the sample was loaded, and after the sample was loaded, the sample was again equilibrated and eluted with PBS at a flow rate of 1 ml/min, and 0.4 ml was collected from each tube. The peak components were analyzed by 12% SDS-PAGE and then combined.

A final anion-exchange purification was performed using an anion-exchange column, HiTrap^{®} Q HP (GE Healthcare). The samples purified in the previous step were diluted 20-fold using pre-cooled 10 mM Tris and then loaded. The column was rinsed first with four column volumes of solution A, and then the sample was loaded at a flow rate of 5 ml/min. After the sample was fully loaded, the column was again rinsed with solution A for about 4 column volumes. When both conductivity and UV₂₈₀ stabilized, a gradient elution was started, setting 0-100% of solution B for 50 min, eluting the sample at a flow rate of 3 ml/min, and collecting the sample when the UV₂₈₀ was significantly elevated, with 1 ml collected from each tube. The peak components were analyzed by 12% SDS-PAGE and then combined. The samples were concentrated using a 10 kDa ultrafiltration tube at 3500×g, 4 °C to 500 µl and the buffer was replaced using PBS buffer and the concentration was determined, then subdivided and stored at -80 °C.

The SDS-PAGE profile of the purified protein was shown in Figure 30: Lane 2: multi-domain bifunctional fusion molecule (VH105-TCR β form) in non-reduced state; Lane 3: marker; Lane 5: multi-domain bifunctional fusion molecule (VH105-TCR β form) in reduced state.

### Example 6

### Preparation of a multi-domain fusion protein complex molecule (VL43-TCR β form) formed by the fusion of the N-terminus of the 1G4 TCR β chain with the antibody light chain variable region

SEQ ID NO: 1 (Figure 1a) is the amino acid sequence of the 1G4 TCR α chain, wherein Thr47 in its TRAC constant region is substituted with Cys162.
SEQ ID NO: 2 (Figure 1b) is the nucleic acid sequence corresponding to SEQ ID NO: 1.
SEQ ID NO: 3 (Figure 2a) is the amino acid sequence of the 1G4 TCR β chain, wherein Ser56 in its TRBC constant region is substituted with Cys168.
SEQ ID NO: 4 (Figure 2b) is the nucleic acid sequence corresponding to SEQ ID NO: 3.
SEQ ID NO: 22 (Figure 12a) is the amino acid sequence of the antibody light chain variable region, wherein Ala43 in the original sequence is substituted with Cys43.
SEQ ID NO: 23 (Figure 12b) is the nucleic acid sequence corresponding to SEQ ID NO: 22.
SEQ ID NO: 20 (Figure 11a) is the amino acid sequence of the antibody heavy chain variable region, wherein Gln105 in the original sequence is substituted with Cys105.
SEQ ID NO: 21 (Figure 11b) is the nucleic acid sequence corresponding to SEQ ID NO: 20.

Figure 27 shows in block diagram form the structure of a multi-domain fusion molecule containing the α-chain shown in SEQ ID NO: 1 and the β-chain shown in SEQ ID NO: 3, and containing the antibody light chain shown in SEQ ID NO: 22 and the antibody heavy chain shown in SEQ ID NO: 20. The antibody light chain is fused to the N-terminus of the TCR β chain shown in SEQ ID NO: 3 via a linker with the sequence shown in SEQ ID NO: 26 (Figure 14a). The linker sequence is GGGGS (SEQ ID NO: 9).

SEQ ID NO: 27 (Figure 14b) is the nucleic acid sequence corresponding to SEQ ID NO: 26.

### Vector construction

The genes SEQ ID NO: 2 (Figure 1b) encoding the 1G4 TCR α-chain and SEQ ID NO: 27 (Figure 14b) encoding the antibody light chain fused to the 1G4 TCR β-chain, and SEQ ID NO: 21 (Figure 1 1b) encoding the antibody heavy chain were respectively cloned into the expression plasmid of pET-28a, which contained the T7 promoter to allow the high-level expression of the target genes in the *E. coli* BL21-DE3 strains.

### Expression

The constructed plasmids were individually transformed into *E. coli* strains BL21-DE3, and kanamycin-resistant monoclonal clones were grown in LB medium (50 µg/ml of kanamycin) at 37°C until the OD₆₀₀ was about 1.0, and then protein expression was induced with 1 mM IPTG. Three hours after induction, cells were collected by centrifugation at 4000 g for 15 min in a Thermo Scientific HERAEUS X1R centrifuge. Using 20 ml of BugBuster Master Mix (Merck Millipore), the organisms were resuspended by vortexing and shaking, and then after shaking and processing at room temperature for 20 min, they were placed in a pre-cooled high-speed centrifuge at 6000 g, 4°C, for 15 min, and then the supernatant was removed. 10 ml of BugBuster Master Mix was added again, the precipitate was resuspended by vortexing and shaking at room temperature for 5 min, then 30 ml of 10-fold diluted BugBuster (Merck Millipore) was added into it again, and the liquid was inverted up and down several times to mix well, then was put into the high-speed centrifuge again at 6000 g, 4 °C, for 15 min, and the supernatant was discarded. 30 ml of 10-fold diluted BugBuster was added, the precipitate was resuspended by vortexing and shaking, and the liquid was put into the high-speed centrifuge at 6000 g, 4°C for 15 min. The supernatant was discarded and the previous step was repeated twice. The supernatant was discarded, 30 ml of PBS was added to resuspend the inclusion bodies and centrifuged at 6000 g, 4°C for 15 min. After discarding the supernatant, 6 M of guanidine hydrochloride was added to dissolve the inclusion bodies. The purified inclusion bodies were serially diluted, then samples were subjected to SDS-PAGE for purity detection and yield estimation, and all the inclusion bodies were subjected to protein quantification, and were subdivided and transferred to -80°C for storage.

### Renaturation

The renaturation buffer for TCR (5 M urea, 100 mM Tris pH 8.1, 0.4 M L-arginine, 2 mM EDTA, 6.5 mM cysteamine, 1.87 mM cystamine) was prepared and pre-cooled to 4°C in advance. About 12 mg of TCR α chain and 11.2 mg of antibody light chain-TCR β chain, and 6 mg of antibody heavy chain inclusion bodies were thawed from the cryopreservation medium. They were added to 6 ml of 6 M guanidine hydrochloride solution, respectively, and DTT at a final concentration of 15 mM was added. The mixture was mixed well, and then placed in a 37°C incubator for 40-min incubation. The incubated TCR α chain and light chain-TCR β chain and antibody heavy chain inclusion bodies were added to the renaturation buffer of the TCR, respectively, and reacted for 30 min in a cold storage. A dialysis bag of 10 kDa was prepared and the reacted renaturation buffer was added into the dialysis bag, then the dialysis bag was put into pre-cooled deionized water and subjected to dialysis overnight in the cold storage. The next day, the dialysis bag was transferred to pre-cooled 10 mM Tris-HCl for dialysis. In the evening, the dialysis bag was again transferred to pre-cooled 10 mM Tris-HCl for dialysis overnight.

### Purification

Soluble and correctly folded multi-domain fusion molecules were separated from misfolding, degradation products and impurities by a 3-step purification method described below.

Anion exchange purification was used first. The renaturated and dialyzed samples were placed in a pre-cooled high-speed centrifuge at 8000×g, 4°C for 15 min to remove the precipitate and the supernatant was again filtered through a 0.45 µm filter membrane. An anion-exchange column, HiTrap^{®} Q HP (GE Healthcare), was used to purify the renaturated samples. The column was rinsed with four column volumes of solution A (10 mM Tris-HCl, pH 8.0) and then the sample was loaded at a flow rate of 5 ml/min. After the sample was completely loaded, the column was again rinsed with solution A for about 4 column volumes, and when the conductivity and UV₂₈₀ both stabilized, a gradient elution was started, setting 0-100% of solution B (1M NaCl+10 mM Tris-HCl, pH 8.0) for 50 min, eluting the sample at a flow rate of 3 ml/min, and starting to collect when the UV₂₈₀ was significantly elevated, 1 ml was collected from each tube. The peak components were analyzed by 12% SDS-PAGE and then combined.

This was followed by purification using molecular sieves (Superdex 75, GE Healthcare). The sample purified in the previous step was concentrated using a 10 kDa ultrafiltration tube at 3500×g, 4°C to 500 µl, followed by molecular sieve purification. The molecular sieves were equilibrated first with deionized water and then equilibrated again with PBS. The sample loop was washed with 500 µl of PBS and the sample was loaded, and after the sample was loaded, the sample was again equilibrated and eluted with PBS at a flow rate of 1 ml/min, and 0.4 ml was collected from each tube. The peak components were analyzed by 12% SDS-PAGE and then combined.

A final anion-exchange purification was performed using an anion-exchange column, HiTrap^{®} Q HP (GE Healthcare). The samples purified in the previous step were diluted 20-fold using pre-cooled 10 mM Tris and then loaded. The column was rinsed first with four column volumes of solution A, and then the sample was loaded at a flow rate of 5 ml/min. After the sample was fully loaded, the column was again rinsed with solution A for about 4 column volumes. When both conductivity and UV₂₈₀ stabilized, a gradient elution was started, setting 0-100% of solution B for 50 min, eluting the sample at a flow rate of 3 ml/min, and collecting the sample when the UV₂₈₀ was significantly elevated, with 1 ml collected from each tube. The peak components were analyzed by 12% SDS-PAGE and then combined. The samples were concentrated using a 10 kDa ultrafiltration tube at 3500×g, 4 °C to 500 µl and the buffer was replaced using PBS buffer and the concentration was determined, then subdivided and stored at -80 °C.

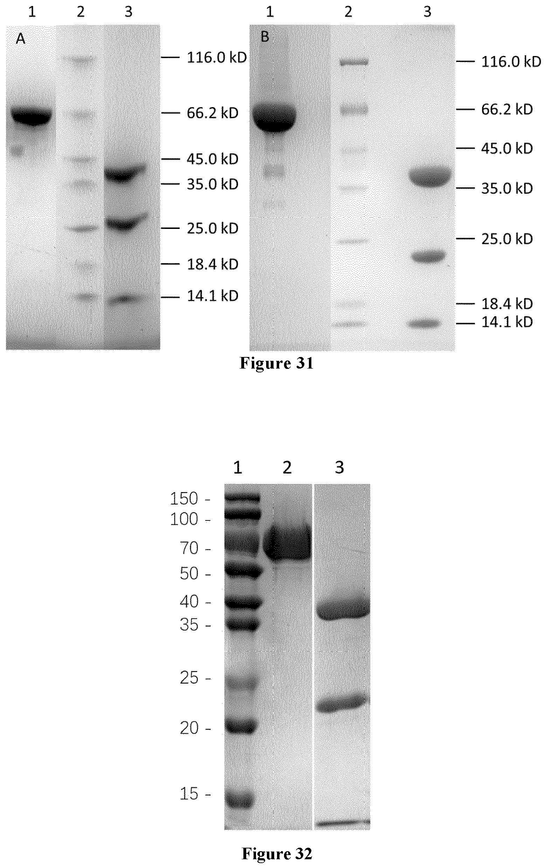

The SDS-PAGE profile of the purified protein was shown in Figure 31 (A): Lane 1: multi-domain bifunctional fusion molecule (VL43-TCR β form) in non-reduced state; Lane 2: marker; Lane 3: multi-domain bifunctional fusion molecule (VL43-TCR β form) in reduced state.

### Example 7

### Preparation of a multi-domain fusion protein complex molecule (VL100-TCR β form) formed by the fusion of the N-terminus of the 1G4 TCR β chain with the antibody light chain variable region

SEQ ID NO: 1 (Figure 1a) is the amino acid sequence of the 1G4 TCR α chain, wherein Thr47 in its TRAC constant region is substituted with Cys162.
SEQ ID NO: 2 (Figure 1b) is the nucleic acid sequence corresponding to SEQ ID NO: 1.
SEQ ID NO: 3 (Figure 2a) is the amino acid sequence of the 1G4 TCR β chain, wherein Ser56 in its TRBC constant region is substituted with Cys168.
SEQ ID NO: 4 (Figure 2b) is the nucleic acid sequence corresponding to SEQ ID NO: 3.
SEQ ID NO: 16 (Figure 9a) is the amino acid sequence of the antibody light chain variable region, wherein Gln100 in the original sequence is substituted with Cys100.
SEQ ID NO: 17 (Figure 9b) is the nucleic acid sequence corresponding to SEQ ID NO: 16.
SEQ ID NO: 14 (Figure 8a) is the amino acid sequence of the antibody heavy chain variable region, wherein Gly44 in the original sequence is substituted with Cys44.
SEQ ID NO: 15 (Figure 8b) is the nucleic acid sequence corresponding to SEQ ID NO: 14.

Figure 28 shows in block diagram form the structure of a multi-domain fusion molecule containing the α-chain shown in SEQ ID NO: 1 and the β-chain shown in SEQ ID NO: 3, and containing the antibody light chain shown in SEQ ID NO: 16 and the antibody heavy chain variable region shown in SEQ ID NO: 14. The antibody light chain is fused to the N-terminus of the TCR β chain shown in SEQ ID NO: 3 via a linker with the sequence shown in SEQ ID NO: 28 (Figure 15a). The linker sequence is GGGGS (SEQ ID NO: 9).

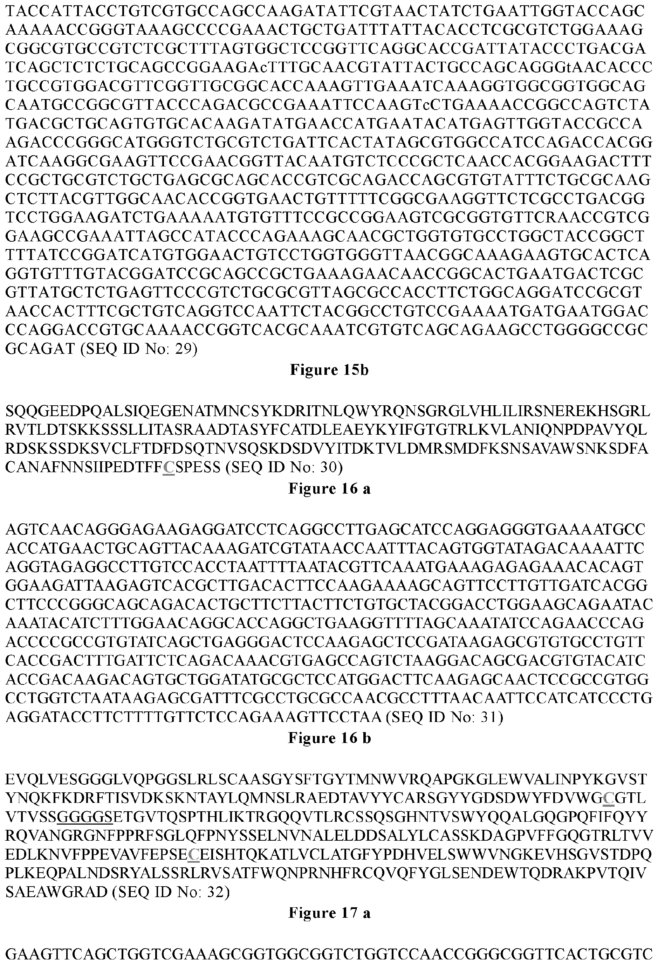

SEQ ID NO: 29 (Figure 15b) is the nucleic acid sequence corresponding to SEQ ID NO: 28.

### Vector construction

The genes SEQ ID NO: 2 (Figure 1b) encoding the 1G4 TCR α-chain and SEQ ID NO: 29 (Figure 15b) encoding the antibody light chain fused to the 1G4 TCR β-chain, and SEQ ID NO: 6 (Figure 3b) encoding the antibody heavy chain were respectively cloned into the expression plasmid of pET-28a, which contained the T7 promoter to allow the high-level expression of the target genes in the *E. coli* BL21-DE3 strains.

### Expression

The constructed plasmids were individually transformed into *E. coli* strains BL21-DE3, and kanamycin-resistant monoclonal clones were grown in LB medium (50 µg/ml of kanamycin) at 37°C until the OD₆₀₀ was about 1.0, and then protein expression was induced with 1 mM IPTG. Three hours after induction, cells were collected by centrifugation at 4000 g for 15 min in a Thermo Scientific HERAEUS X1R centrifuge. Using 20 ml of BugBuster Master Mix (Merck Millipore), the organisms were resuspended by vortexing and shaking, and then after shaking and processing at room temperature for 20 min, they were placed in a pre-cooled high-speed centrifuge at 6000 g, 4°C, for 15 min, and then the supernatant was removed. 10 ml of BugBuster Master Mix was added again, the precipitate was resuspended by vortexing and shaking at room temperature for 5 min, then 30 ml of 10-fold diluted BugBuster (Merck Millipore) was added into it again, and the liquid was inverted up and down several times to mix well, then was put into the high-speed centrifuge again at 6000 g, 4 °C, for 15 min, and the supernatant was discarded. 30 ml of 10-fold diluted BugBuster was added, the precipitate was resuspended by vortexing and shaking, and the liquid was put into the high-speed centrifuge at 6000 g, 4°C for 15 min. The supernatant was discarded and the previous step was repeated twice. The supernatant was discarded, 30 ml of PBS was added to resuspend the inclusion bodies and centrifuged at 6000 g, 4°C for 15 min. After discarding the supernatant, 6 M of guanidine hydrochloride was added to dissolve the inclusion bodies. The purified inclusion bodies were serially diluted, then samples were subjected to SDS-PAGE for purity detection and yield estimation, and all the inclusion bodies were subjected to protein quantification, and were subdivided and transferred to -80°C for storage.

### Renaturation

The renaturation buffer for TCR (5 M urea, 100 mM Tris pH 8.1, 0.4 M L-arginine, 2 mM EDTA, 6.5 mM cysteamine, 1.87 mM cystamine) was prepared and pre-cooled to 4°C in advance. About 12 mg of TCR α chain and 11.2 mg of antibody light chain-TCR β chain, and 6 mg of antibody heavy chain inclusion bodies were thawed from the cryopreservation medium. They were added to 6 ml of 6 M guanidine hydrochloride solution, respectively, and DTT at a final concentration of 15 mM was added. The mixture was mixed well, and then placed in a 37°C incubator for 40-min incubation. The incubated TCR α chain and light chain-TCR β chain and antibody heavy chain inclusion bodies were added to the renaturation buffer of the TCR, respectively, and reacted for 30 min in a cold storage. A dialysis bag of 10 kDa was prepared and the reacted renaturation buffer was added into the dialysis bag, then the dialysis bag was put into pre-cooled deionized water and subjected to dialysis overnight in the cold storage. The next day, the dialysis bag was transferred to pre-cooled 10 mM Tris-HCl for dialysis. In the evening, the dialysis bag was again transferred to pre-cooled 10 mM Tris-HCl for dialysis overnight.

### Purification

Soluble and correctly folded multi-domain fusion molecules were separated from misfolding, degradation products and impurities by a 3-step purification method described below.

Anion exchange purification was used first. The renaturated and dialyzed samples were placed in a pre-cooled high-speed centrifuge at 8000×g, 4°C for 15 min to remove the precipitate and the supernatant was again filtered through a 0.45 µm filter membrane. An anion-exchange column, HiTrap^{®} Q HP (GE Healthcare), was used to purify the renaturated samples. The column was rinsed with four column volumes of solution A (10 mM Tris-HCl, pH 8.0) and then the sample was loaded at a flow rate of 5 ml/min. After the sample was completely loaded, the column was again rinsed with solution A for about 4 column volumes, and when the conductivity and UV₂₈₀ both stabilized, a gradient elution was started, setting 0-100% of solution B (1M NaCl+10 mM Tris-HCl, pH 8.0) for 50 min, eluting the sample at a flow rate of 3 ml/min, and starting to collect when the UV₂₈₀ was significantly elevated, 1 ml was collected from each tube. The peak components were analyzed by 12% SDS-PAGE and then combined.

This was followed by purification using molecular sieves (Superdex 75, GE Healthcare). The sample purified in the previous step was concentrated using a 10 kDa ultrafiltration tube at 3500×g, 4°C to 500 µl, followed by molecular sieve purification. The molecular sieves were equilibrated first with deionized water and then equilibrated again with PBS. The sample loop was washed with 500 µl of PBS and the sample was loaded, and after the sample was loaded, the sample was again equilibrated and eluted with PBS at a flow rate of 1 ml/min, and 0.4 ml was collected from each tube. The peak components were analyzed by 12% SDS-PAGE and then combined.

A final anion-exchange purification was performed using an anion-exchange column, HiTrap^{®} Q HP (GE Healthcare). The samples purified in the previous step were diluted 20-fold using pre-cooled 10 mM Tris and then loaded. The column was rinsed first with four column volumes of solution A, and then the sample was loaded at a flow rate of 5 ml/min. After the sample was fully loaded, the column was again rinsed with solution A for about 4 column volumes. When both conductivity and UV₂₈₀ stabilized, a gradient elution was started, setting 0-100% of solution B for 50 min, eluting the sample at a flow rate of 3 ml/min, and collecting the sample when the UV₂₈₀ was significantly elevated, with 1 ml collected from each tube. The peak components were analyzed by 12% SDS-PAGE and then combined. The samples were concentrated using a 10 kDa ultrafiltration tube at 3500×g, 4 °C to 500 µl and the buffer was replaced using PBS buffer and the concentration was determined, then subdivided and stored at -80 °C.

The SDS-PAGE profile of the purified protein was shown in Figure 31 (B): Lane 1: multi-domain bifunctional fusion molecule (VL100-TCR β form) in non-reduced state; Lane 2: marker; Lane 3: multi-domain bifunctional fusion molecule (VL100-TCR β form) in reduced state.

### Example 8

### Preparation of a multi-domain bifunctional fusion molecule (VH105-TCR β form) formed by the fusion of the N-terminus of the AFP TCR β chain with the antibody heavy chain variable region

SEQ ID NO: 30 (Figure 16a) is the amino acid sequence of the AFP TCR α chain, wherein Pro88 in its TRAC constant region is substituted with Cys200.
SEQ ID NO: 31 (Figure 16b) is the nucleic acid sequence corresponding to SEQ ID NO: 30.
SEQ ID NO: 32 (Figure 117) is the amino acid sequence (Gln105Cys) obtained by fusing the N-terminus of the AFP TCR β chain with the antibody heavy chain variable region, and wherein Ala18 in its TRBC constant region is substituted with Cys257.
SEQ ID NO: 33 (Figure 17b) is the nucleic acid sequence corresponding to SEQ ID NO: 32.

The AFPTCR in this embodiment can bind to the FMNKFIYEI (SEQ ID NO: 38) short peptide presented on the HLA-A*0201 molecule.

### Vector construction

The genes SEQ ID NO: 31 (Figure 16b) encoding the AFP TCR α-chain and SEQ ID NO: 33 (Figure 17b) encoding the antibody heavy chain fused to the AFP TCR β-chain, and SEQ ID NO: 23 (Figure 12b) encoding the antibody light chain were respectively cloned into the expression plasmid of pET-28a, which contained the T7 promoter to allow the high-level expression of the target genes in the *E. coli* BL21-DE3 strains.

### Expression

The constructed plasmids were individually transformed into *E. coli* strains BL21-DE3, and kanamycin-resistant monoclonal clones were grown in LB medium (50 µg/ml of kanamycin) at 37°C until the OD₆₀₀ was about 1.0, and then protein expression was induced with 1 mM IPTG. Three hours after induction, cells were collected by centrifugation at 4000 g for 15 min in a Thermo Scientific HERAEUS X1R centrifuge. Using 20 ml of BugBuster Master Mix (Merck Millipore), the organisms were resuspended by vortexing and shaking, and then after shaking and processing at room temperature for 20 min, they were placed in a pre-cooled high-speed centrifuge at 6000 g, 4°C, for 15 min, and then the supernatant was removed. 10 ml of BugBuster Master Mix was added again, the precipitate was resuspended by vortexing and shaking at room temperature for 5 min, then 30 ml of 10-fold diluted BugBuster (Merck Millipore) was added into it again, and the liquid was inverted up and down several times to mix well, then was put into the high-speed centrifuge again at 6000 g, 4 °C, for 15 min, and the supernatant was discarded. 30 ml of 10-fold diluted BugBuster was added, the precipitate was resuspended by vortexing and shaking, and the liquid was put into the high-speed centrifuge at 6000 g, 4°C for 15 min. The supernatant was discarded and the previous step was repeated twice. The supernatant was discarded, 30 ml of PBS was added to resuspend the inclusion bodies and centrifuged at 6000 g, 4°C for 15 min. After discarding the supernatant, 6 M of guanidine hydrochloride was added to dissolve the inclusion bodies. The purified inclusion bodies were serially diluted, then samples were subjected to SDS-PAGE for purity detection and yield estimation, and all the inclusion bodies were subjected to protein quantification, and were subdivided and transferred to -80°C for storage.

### Renaturation

The renaturation buffer for TCR (5 M urea, 100 mM Tris pH 8.1, 0.4 M L-arginine, 2 mM EDTA, 6.5 mM cysteamine, 1.87 mM cystamine) was prepared and pre-cooled to 4°C in advance. About 12 mg of TCR α chain and 11.2 mg of antibody heavy chain-TCR β chain, and 6 mg of antibody light chain inclusion bodies were thawed from the cryopreservation medium. They were added to 6 ml of 6 M guanidine hydrochloride solution, respectively, and DTT at a final concentration of 15 mM was added. The mixture was mixed well, and then placed in a 37°C incubator for 40-min incubation. The incubated TCR α chain and heavy chain-TCR β chain and antibody light chain inclusion bodies were added to the renaturation buffer of the TCR, respectively, and reacted for 30 min in a cold storage. A dialysis bag of 10 kDa was prepared and the reacted renaturation buffer was added into the dialysis bag, then the dialysis bag was put into pre-cooled deionized water and subjected to dialysis overnight in the cold storage. The next day, the dialysis bag was transferred to pre-cooled 10 mM Tris-HCl for dialysis. In the evening, the dialysis bag was again transferred to pre-cooled 10 mM Tris-HCl for dialysis overnight.

### Purification

Soluble and correctly folded multi-domain fusion molecules were separated from misfolding, degradation products and impurities by a 2-step purification method described below.

Anion exchange purification was used first. The dialyzed samples were placed in a pre-cooled high-speed centrifuge at 8000×g, 4°C for 15 min to remove the precipitate and the supernatant was again filtered through a 0.45 µm filter membrane. An anion-exchange column, HiTrap^{®} Q HP (GE Healthcare), was used to purify the renaturated samples. The column was rinsed with four column volumes of solution A (10 mM Tris-HCl, pH 8.0) and then the sample was loaded at a flow rate of 5 ml/min. After the sample was completely loaded, the column was again rinsed with solution A for about 4 column volumes, and when the conductivity and UV₂₈₀ both stabilized, a gradient elution was started, setting 0-100% of solution B (1M NaCl+10 mM Tris-HCl, pH 8.0) for 50 min, eluting the sample at a flow rate of 3 ml/min, and starting to collect when the UV₂₈₀ was significantly elevated, 1 ml was collected from each tube. The peak components were analyzed by 12% SDS-PAGE and then combined.

This was followed by purification using molecular sieves (Superdex 75, GE Healthcare). The sample purified in the previous step was concentrated using a 10 kDa ultrafiltration tube at 3500×g, 4°C to 500 µl, followed by molecular sieve purification. The molecular sieves were equilibrated first with deionized water and then equilibrated again with PBS. The sample loop was washed with 500 µl of PBS and the sample was loaded, and after the sample was loaded, the sample was again equilibrated and eluted with PBS at a flow rate of 1 ml/min, and 0.4 ml was collected from each tube. The peak components were analyzed by 12% SDS-PAGE and then combined, and replaced the buffer with PBS buffer. The concentrations were measured and then the components were subdivided and stored at -80 °C.

The SDS-PAGE profile of the purified protein was shown in Figure 32: Lane 1: marker; Lane 2: multi-domain bifunctional fusion molecule (VH105-TCR β form) in non-reduced state; Lane 3: multi-domain bifunctional fusion molecule (VH105-TCR β form) in reduced state.

### Example 9

### Properties of multi-domain bifunctional fusion molecules in the form of VH-TCR β or VL-TCR β

The ability of bifunctional fusion molecules to redirect T cells to specifically kill tumor cells has been reported in several studies. The basic principle is that they can mimic the key signals of T cell activation to exert effector functions. On the one hand, they can recognize the pMHC complex on the surface of tumor cells through their specific TCRs with high affinity; and on the other hand, they can activate the downstream signaling pathways of T cell activation through the CD3 antibody end, so as to direct the T cells to specifically kill the tumor cells.

The following embodiments demonstrate that the multi-domain bifunctional fusion molecules of the present invention are capable of redirecting CD8⁺ T cells to kill target cell lines. This assay is a colorimetric assay alternative to the 51Cr release cytotoxicity assay that quantifies lactate dehydrogenase (LDH) released upon cell lysis. A 30-minute coupled enzyme reaction was used to detect LDH released in the medium, and in the enzyme reaction LDH converts a tetrazolium salt (INT) to a red colored formanzan. The amount of red product generated is proportional to the number of cells lysed. Data for visible light absorption values at 490 nm can be collected using a standard 96-well plate reader.

Those skilled in the art are familiar with methods for detecting cell function using LDH release assays. The target cell lines selected for LDH experiments in this embodiment are MDA-MB-231 (NY-ESO-1 positive, A2 positive, breast cancer cell line), MDA-MB-231 (NY-ESO-1 negative, A2 positive, breast cancer cell line) and NCI-H1299-A2 (NY-ESO-1 positive, A2 positive, lung cancer cell line), NCI-H1299 (NY-ESO-1 positive, A2 negative, lung cancer cell line), HepG2 (A2 positive, AFP positive, liver cancer cell line), NCI-H1299-A2 (A2 positive, AFP negative, lung cancer cell line), and HepG2 (A2 positive, gp100 positive, liver cancer cell line), and NCI-H1299 (A2 negative, gp100 positive, lung cancer cell line).

### 9.1 Multi-domain bifunctional fusion molecules of VH44-TCR β-1G4, VH105-TCR β-1G4 redirect CD8⁺ T-cells to kill the MDA-MB-231 (NY-ESO-1 positive, A2 positive) breast cancer cell line and the NCI-H1299 (NY-ESO-1 positive, A2 positive) lung cancer cell line

CD8⁺ T cells were used as effector cells, and MDA-MB-231 (NY-ESO-1 positive, A2 positive, breast cancer cell line) and NCI-H1299 (NY-ESO-1 positive, A2 positive, lung cancer cell line) were used as target cells, and the E : T ratio selected was 5:1. Both the desired effector and target cells were diluted well in culture medium according to the desired number of cells, i.e. the cell density of the desired effector cells was 1×10⁶ /ml, while the cell density of the desired target cells was 2×10⁵ /ml. The MDA-MB-231 (NY-ESO-1 negative, A2 positive, breast cancer cell line) and NCI-H1299 (NY-ESO-1 positive, A2 negative, lung cancer cell line) were also used as negative control target cells.

When the NCI-H1299 (NY-ESO-1 positive, A2 positive, lung cancer cell line) was used as a target cell, the proteins of the VH44-TCR β-1G4, or VH105-TCR β-1G4 multi-domain bifunctional fusion molecule and the control molecule (VH-TCR β-1G4 dimeric fusion molecule without an immune effect) were diluted with the culture medium to 4×10⁻⁷ M, 4×10⁻⁸ M, 4×10⁻⁹ M, 4×10⁻¹⁰ M, 4×10⁻¹¹ M, and 4×10⁻¹² M.

When the MDA-MB-231 (NY-ESO-1 positive, A2 positive, breast cancer cell line) was used as a target cell, the proteins of the VH44-TCR β-1G4, or VH105-TCR β-1G4 multi-domain bifunctional fusion molecule and the control molecule (VH-TCR β-1G4 dimeric fusion molecule without an immune effect) were diluted with culture medium to 4×10⁻⁹ M, 4×10⁻¹⁰ M, 4×10⁻¹¹ M, 4×10⁻¹² M, and 4×10⁻¹³ M.

A 96-well round-bottom plate was prepared and 50 µl of effector cells, 50 µl of multi-domain bifunctional fusion molecule proteins and 100 µl of target cells were added to the wells, and the target cell maximal lysis wells, the target cell spontaneous wells, the effector cell spontaneous wells, the medium spontaneous wells, and the medium plus the lysate spontaneous wells were set up with a final volume of 200 µl for each well, and each of them has three duplicate wells. The 96-well round-bottomed plates were incubated in a cell culture incubator at 37°C, 5% CO₂ for 24 h. After that, the 96-well round-bottomed plates were put into a centrifuge at 250×g for 4 min. 50 µl of supernatant was taken out to the 96-well flat-bottomed plates and 50 µl of the substrate solution was added, and the reaction was carried out at ambient temperature under the protection from light for 30 min. After the reaction was finished, 50 µl of termination solution was added and the absorption value was measured at 490 nm by a microplate reader immediately. The specific killing efficiency was calculated according to the instruction manual = (experimental wells - effector cell spontaneous wells - target cell spontaneous wells) / (target cell maximum lysis wells - target cell spontaneous wells).

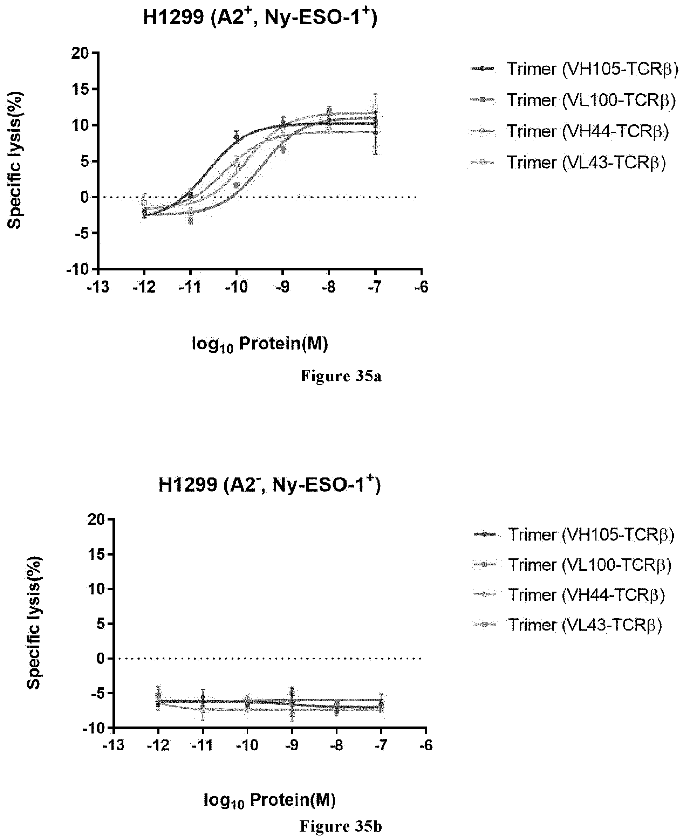

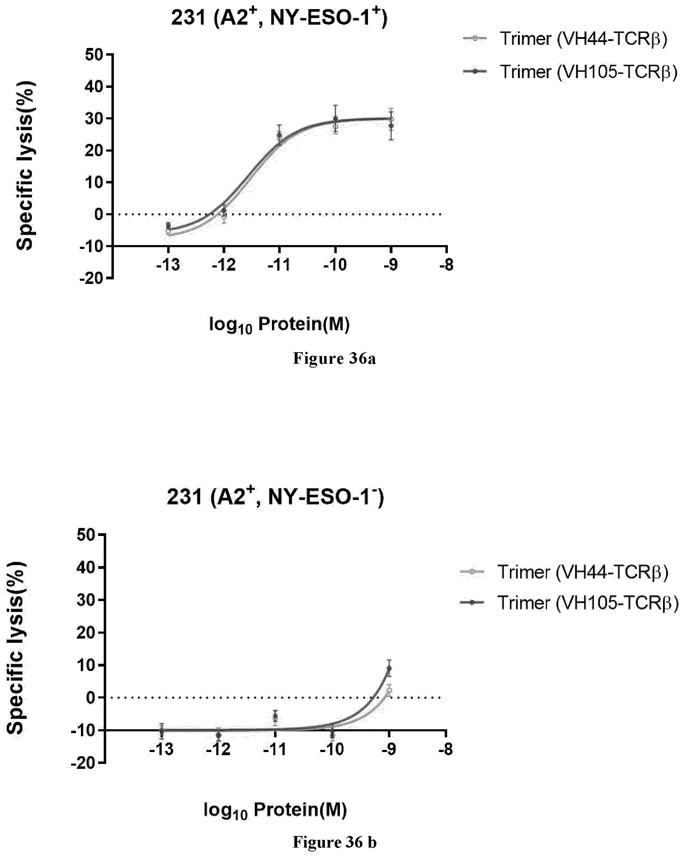

The results, as shown in Figures 35 and 36, showed that multi-domain bifunctional molecules in the form of VH44-TCR β-1G4 and VH105-TCR β-1G4 were able to mediate CD8⁺ T cell killing on the MDA-MB-231 (NY-ESO-1 positive, A2 positive, breast cancer cell line) and the NCI-H1299 (NY-ESO-1 positive, A2 positive, lung cancer cell line), but could not kill the MDA-MB-231 (NY-ESO-1 negative, A2 positive, breast cancer cell line) and the NCI-H1299 (NY-ESO-1 positive, A2 negative, lung cancer cell line). The EC50 values were determined by graphing the percentage of killing observed in each well against the corresponding protein concentration using Graphpad Prism software. The EC50 values were 2.9e⁻¹² for the VH44-TCR β-1G4 form against the MDA-MB-231 (NY-ESO-1 positive, A2 positive, breast cancer cell line) and 5.7e⁻¹¹ for the NCI-H1299 (NY-ESO-1 positive, A2 positive, lung cancer cell line); 2.7e⁻¹² for the VH105-TCR β-1G4 form against the MDA-MB-231 (NY-ESO-1 positive, A2 positive, breast cancer cell line) and 2.4e⁻¹¹ for the NCI-H1299 (NY-ESO-1 positive, A2 positive, lung cancer cell line).

### 9.2 Multi-domain bifunctional fusion molecules in the form of VL43-TCR β-1G4, VL100-TCR β-1G4 redirect CD8⁺ cells to kill NCI-H1299 (NY-ESO-1 positive, A2 positive, lung cancer cell line)

CD8⁺ T cells were used as effector cells, and NCI-H1299 (NY-ESO-1 positive, A2 positive, lung cancer cell line) was used as a target cell, and the E : T ratio selected was 5:1. Both the desired effector and target cells were diluted well in culture medium according to the desired number of cells, i.e., the cell density of the desired effector cells was 1×10⁶ /ml, while the cell density of the desired target cells was 2×10⁵ /ml. The NCI-H1299 (NY-ESO-1 positive, A2 negative, lung cancer cell line) was also used as a negative control target cell.

The protein concentrations of the multi-domain bifunctional fusion molecules in the form of VL43-TCR β-1G4, VL100-TCR β-1G4 and the control molecule (VL-TCR β-1G4 dimeric fusion molecule without an immune effect) were diluted with the culture medium to 4×10⁻⁷ M, 4×10⁻⁸ M, 4×10⁻⁹ M, 4×10⁻¹⁰ M, 4×10⁻¹¹ M, and 4×10⁻¹² M.

A 96-well round-bottom plate was prepared and 50 µl of effector cells, 50 µl of multi-domain bifunctional fusion molecule proteins and 100 µl of target cells were added to the wells, and the target cell maximal lysis wells, the target cell spontaneous wells, the effector cell spontaneous wells, the medium spontaneous wells, and the medium plus the lysate spontaneous wells were set up with a final volume of 200 µl for each well, and each of them has three duplicate wells. The 96-well round-bottomed plates were incubated in a cell culture incubator at 37°C, 5% CO₂ for 24 h. After that, the 96-well round-bottomed plates were put into a centrifuge at 250×g for 4 min. 50 µl of supernatant was taken out to the 96-well flat-bottomed plates and 50 µl of the substrate solution was added, and the reaction was carried out at ambient temperature under the protection from light for 30 min. After the reaction was finished, 50 µl of termination solution was added and the absorption value was measured at 490 nm by a microplate reader immediately. The specific killing efficiency was calculated according to the instruction manual = (experimental wells - effector cell spontaneous wells - target cell spontaneous wells) / (target cell maximum lysis wells - target cell spontaneous wells).

The results, as shown in Figure 35, showed that multi-domain bifunctional molecules in the form of VL43-TCR β-1G4 and VL100-TCR β-1G4 were able to mediate CD8⁺ T cell killing on the NCI-H1299 (NY-ESO-1 positive, A2 positive, lung cancer cell line), but could not kill the NCI-H1299 (NY-ESO-1 positive, A2 negative, lung cancer cell line). The EC50 values were determined by graphing the percentage of killing observed in each well against the corresponding protein concentration using Graphpad Prism software. The EC50 values were 1.7e⁻¹⁰ for the VL44-TCR β-1G4 form against the NCI-H1299 (NY-ESO-1 positive, A2 positive, lung cancer cell line); and 3.4e⁻¹⁰ for the VL100-TCR β-1G4 form against the NCI-H1299 (NY-ESO-1 positive, A2 positive, lung cancer cell line).

### 9.3 Multi-domain bifunctional fusion molecules in the form of VH105-TCR β-AFP redirect CD8⁺ cells to kill HepG2 (A2 positive, AFP positive, liver cancer cell line)

CD8⁺ T cells were used as effector cells, and HepG2 (A2 positive, AFP positive, liver cancer cell line) was used as a target cell, and the E : T ratio selected was 1: 1. Both the desired effector and target cells were diluted well in culture medium according to the desired number of cells, i.e., the cell density of the desired effector cells was 2×10⁵ /ml, while the cell density of the desired target cells was 2×10⁵ /ml. The NCI-H1299-A2 (A2 positive, AFP negative, lung cancer cell line) was also used as a negative control target cell.

The protein concentrations of the multi-domain bifunctional fusion molecules in the form of VH105-TCR β-AFP were diluted with the culture medium to 4×10⁻⁷ M, 4×10⁻⁸ M, 4×10⁻⁹ M, 4×10⁻¹⁰ M, 4×10⁻¹¹ M, 4×10⁻¹² M, and 4×10⁻¹³ M.

A 96-well round-bottom plate was prepared and 50 µl of effector cells, 50 µl of multi-domain bifunctional fusion molecule proteins and 100 µl of target cells were added to the wells, and the target cell maximal lysis wells, the target cell spontaneous wells, the effector cell spontaneous wells, the medium spontaneous wells, and the medium plus the lysate spontaneous wells were set up with a final volume of 200 µl for each well, and each of them has three duplicate wells. The 96-well round-bottomed plates were incubated in a cell culture incubator at 37°C, 5% CO₂ for 24 h. After that, the 96-well round-bottomed plates were put into a centrifuge at 250×g for 4 min. 50 µl of supernatant was taken out to the 96-well flat-bottomed plates and 50 µl of the substrate solution was added, and the reaction was carried out at ambient temperature under the protection from light for 30 min. After the reaction was finished, 50 µl of termination solution was added and the absorption value was measured at 490 nm by a microplate reader immediately. The specific killing efficiency was calculated according to the instruction manual = (experimental wells - effector cell spontaneous wells - target cell spontaneous wells) / (target cell maximum lysis wells - target cell spontaneous wells).

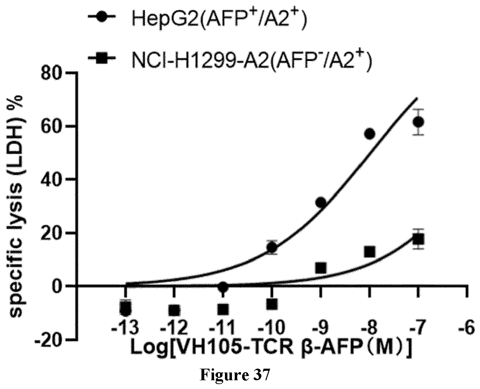

The results, as shown in Figure 37, showed that the multi-domain bifunctional molecules in the form of VH105-TCR β-AFP were able to mediate CD8⁺ T cell efficient killing on the HepG2 (A2 positive, AFP positive, liver cancer cell line), but the killing efficiency on the NCI-H1299-A2 (A2 positive, AFP negative, lung cancer cell line) is low; when the protein was at a safe dose (<1×10⁻⁹ M), it did not kill negative target cells. The EC50 values were determined by graphing the percentage of killing observed in each well against the corresponding protein concentration using Graphpad Prism software. The EC50 values were 1.05e⁻⁸ for the VH105-TCR β-AFP form against the HepG2 (A2 positive, AFP positive, liver cancer cell line); and 2.7e⁻⁶ against the NCI-H1299-A2 (A2 positive, AFP negative, lung cancer cell line).

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents also fall in the scope of the claims appended to this application.

## Claims

1. A fusion protein which comprises a polypeptide with a structure from the N-terminus to the C-terminus as shown in Formula Ia or Ib:
A1-L-B (Ia);
B-L-A1 (Ib);
wherein,
element A1 is a polypeptide molecule without an immune effect;
element B is a polypeptide receptor molecule or component thereof, or fragment thereof that specifically binds a pMHC epitope;
element L is a flexible linker; wherein the flexible linker is optional;
"-" is a covalent bond.

2. A fusion protein, the structure of which from the N-terminus to the C-terminus is as shown in Formula Ia or Ib:
A1-L-B (Ia);
B-L-A1 (Ib);
wherein,
element A1 is a polypeptide molecule without an immune effect;
element B is a polypeptide receptor molecule or component thereof, or fragment thereof that specifically binds a pMHC epitope;
element L is a flexible linker;
"-" is a covalent bond.

3. The fusion protein of claim 1 or 2, wherein the element A1 is a heavy chain variable region of an antibody or a light chain variable region of an antibody.

4. The fusion protein of claim 3, wherein the amino acid sequence of the heavy chain variable region of the antibody comprises a sequence as shown in SEQ ID NO: 14, SEQ ID NO: 20, or SEQ ID NO: 5.

5. The fusion protein of claim 3, wherein the amino acid sequence of the light chain variable region of the antibody comprises a sequence as shown in SEQ ID NO: 16, SEQ ID NO: 22 or SEQ ID NO: 10.

6. The fusion protein of claim 1 or 2, wherein the element B is selected from the group consisting of: a TCR molecule, a single chain αβTCR, a TCRα chain/TCRβ chain heterodimer complex molecule, an antibody molecule Fab complex molecule, a single chain antibody molecule, and combinations thereof.

7. The fusion protein of claim 6, wherein the amino acid sequence of TCRα chain comprises a sequence as shown in SEQ ID NO: 1 and the amino acid sequence of TCRβ chain comprises a sequence as shown in SEQ ID NO: 3.

8. The fusion protein of claim 7, wherein the element A1 is linked to the TCRβ chain by a flexible linker L.

9. The fusion protein of claim 1 or 2, wherein the element L comprises a structure (GGGGS)n, wherein n is a positive integer from 1-5, preferably comprising a sequence as shown in SEQ ID NO: 9.

10. The fusion protein of claim 1, wherein the amino acid sequence of the fusion protein comprises a sequence as shown in SEQ ID NO: 18, SEQ ID NO: 24, SEQ ID NO: 26, SEQ ID NO: 28, SEQ ID NO: 7, SEQ ID NO: 12, or SEQ ID NO: 32, or a sequence that has at least 95% or more, at least 98% or more, at least 99% or more, at least 99.9% or more identity with the sequence as shown in SEQ ID NO: 18, SEQ ID NO: 24, SEQ ID NO: 26, SEQ ID NO: 28, SEQ ID NO: 7, SEQ ID NO: 12, or SEQ ID NO: 32.

11. A fusion protein complex molecule which has a structure from the N-terminus to the C-terminus as shown in Formula Ic or Id:
A2...A1-L-B (Ic);
B-L-A1...A2 (Id);
wherein,
elements A1 and A2 are each independently a polypeptide molecule without an immune effect;
element B is a polypeptide receptor molecule or component thereof, or fragment thereof that specifically binds a pMHC epitope; and
element L is a flexible linker;
"-" is a covalent bond;
"..." is a disulfide bond or non-covalent interaction between protein domains.

12. The fusion protein complex molecule of claim 11, wherein the element A1 is a heavy chain variable region of the antibody and the element A2 is a light chain variable region of the antibody; or, the element A1 is a light chain variable region of the antibody and the element A2 is a heavy chain variable region of the antibody.

13. The fusion protein complex molecule of claim 12, wherein the heavy chain variable region of the antibody comprises a sequence as shown in SEQ ID NO: 14, SEQ ID NO: 20, or SEQ ID NO: 5.

14. The fusion protein complex molecule of claim 12, wherein the light chain variable region of the antibody comprises a sequence as shown in SEQ ID NO: 16, SEQ ID NO: 22, or SEQ ID NO: 10.

15. The fusion protein complex molecule of claim 11, wherein the element B is selected from the group consisting of: a TCR molecule, a single chain αβTCR, a TCRα chain/TCRβ chain heterodimer complex molecule, an antibody molecule Fab complex molecule, a single-chain antibody molecule, and combinations thereof.

16. The fusion protein complex molecule of claim 15, wherein the amino acid sequence of the TCRα chain comprises a sequence as shown in SEQ ID NO: 1 and the amino acid sequence of the TCRβ chain comprises a sequence as shown in SEQ ID NO: 3.

17. The fusion protein complex molecule of claim 16, wherein the element A1 is linked to the TCRβ chain via a flexible linker L.

18. The fusion protein complex molecule of claim 11, wherein the element L comprises a structure (GGGGS)n, wherein n is a positive integer from 1-5, preferably comprising a sequence as shown in SEQ ID NO: 9.

19. A polynucleotide encoding the fusion protein of any one of claims 1 to 10 or the fusion protein complex molecule of any one of claims 11 to 18.

20. An expression vector comprising the polynucleotide of claim 19.

21. A host cell comprising the expression vector of claim 20 or the polynucleotide of claim 19.

22. A method of generating the fusion protein of any one of claims 1 to 10 or the fusion protein complex molecule of any one of claims 11 to 18, which comprises the steps of:
(a) culturing the host cell of claim 21 under conditions suitable for the production of the fusion protein of any one of claims 1 to 10 or the fusion protein complex molecule of any one of claims 11 to 18, thereby obtaining a culture containing the fusion protein or the fusion protein complex molecule;
(b) isolating or recovering the fusion protein or fusion protein complex molecule from the culture; and
(c) optionally, purifying and/or modifying the fusion protein or fusion protein complex molecule obtained in step (b).

23. An immunoconjugate comprising:
(a) the fusion protein of any one of claims 1 to 10 or the fusion protein complex molecule of any one of claims 11 to 18; and
(b) a coupling moiety selected from the group consisting of: detectable marker, drug, toxin, cytokine, radionuclide, enzyme, gold nanoparticle/nanorod, magnetic nanoparticle, viral capsid protein or VLP, and combinations thereof.

24. A pharmaceutical composition comprising the fusion protein of any one of claims 1 to 10, the fusion protein complex molecule of any one of claims 11 to 18, the polynucleotide of claim 19, the expression vector of claim 20, the host cell of claim 21, or the immunoconjugate of claim 23, and combinations thereof; and a pharmaceutically acceptable carrier.

25. A recombinant protein comprising
(i) the fusion protein of any one of claims 1 to 10, or the fusion protein complex molecule of any one of claims 11 to 18; and
(ii) an optional tag sequence to assist in expression and/or purification.

26. Use of the fusion protein of any one of claims 1 to 10, the fusion protein complex molecule of any one of claims 11 to 18, the polynucleotide of claim 19, the expression vector of claim 20, the host cell of claim 21, the immunoconjugate of claim 23, the pharmaceutical composition of claim 24, or the recombinant protein of claim 25, in the manufacture of a medicament for preventing and/or treating a tumor in a subj ect.

27. Use of claim 26, wherein the tumor comprises breast cancer, lung cancer, liver cancer, and combinations thereof.

28. Use of claim 26, wherein the subject is a mammal, preferably a human.
